# EUROPEAN PATENT SPECIFICATION

(11) **EP 3 352 840 B1**
(45) Date of publication and mention of the grant of the patent: **20.10.2021**
(21) Application number: 16774805.2
(22) Date of filing: 22.09.2016
(51) Int. Cl.: A61M 39/10, A61M 39/12

(54) **A DUAL PORT TUBE FOR SUCTION AND FEEDING SYSTEM, METHOD, AND APPARATUS**
DOPPELANSCHLUSSROHR FÜR SAUG- UND SPEISESYSTEM, VERFAHREN UND VORRICHTUNG
TUBE À DEUX ORIFICES POUR SYSTÈME, PROCÉDÉ ET APPAREIL D'ASPIRATION ET D'ALIMENTATION

(30) Priority: 22.09.2015 US 201562221845 P
(43) Date of publication of application: 01.08.2018
(73) Proprietor: Corpak Medsystems, Inc., Buffalo Grove, Illinois 60089 (US)
(72) Inventor: NORDQUIST, Jeffrey S., Barrington, Illinois 60010 (US); KOELPER, Crystal, Buffalo Grove, Illinois 60089 (US); PURNELL, Shawn, Sandy Springs, GA 30328 (US); DAURELLE, Bernard, 13007 Marseille (FR)
(74) Representative: Dehns
(86) International application number: PCT/US2016/053075
(87) International publication number: WO 2017/053546

(56) References cited:
- US-A- 4 416 273
- US-A- 4 573 965
- US-A- 4 735 607
- US-A- 4 850 350
- US-A1- 2006 005 841
- US-A1- 2006 027 270
- US-A1- 2006 129 092
- US-A1- 2010 168 718
- US-B1- 6 579 254

## Description

### TECHNICAL FIELD

The present disclosure relates in general to a system, method, and apparatus for administering fluids to body cavities, suctioning or aspirating fluids from body cavities, performing gastric decompression, and containing gastric reflux.

### BACKGROUND

The use of connector ports is commonly required in various medical systems that utilize multiple components to deliver or remove one or more fluids to a patient or other person or animal being treated. One example of such a system is an enteral feeding system in which fluid nutrient formula or the like (e.g., a medication, flushing solution, stomach irrigation, additional nutrient formula) is delivered via a series of tubing segments to a patient. Connectors can also be utilized for continuous or intermittent suctioning, for example, to drain gastric contents or refluxed intestinal secretions, collect stomach aspirate gastric lavage, or decompress gasses from the patient's gastrointestinal tract. In such systems, it may be desirable to remove and collect stomach fluids before, after, or intermittently while fluids are delivered to the patient. The current systems and methods employed in the prior art for medical tubing systems for feeding, suctioning or aspirating fluids, gastric decompression, residual volume measurement, and/or medicating patients may be improved upon as presently disclosed.

Related art includes US 2006/0129092 A1 which discloses a single lumen adapter for an automatic valve, and US 4735607 A which discloses a nasogastric tube anti-reflux valve.

### SUMMARY

The present disclosure provides a new and innovative system, method, and apparatus for dual port tube suction and feeding.

In one aspect, the present invention provides a Y-connector for use in a gastric system, as claimed in claim 1.

In an embodiment, the present invention provides a gastric tubing system including the Y-connector of claim 1 and a multiple lumen tube. The multiple lumen tube has an open proximal end and a closed distal end. The open proximal end of the multiple lumen tube is adapted to couple to the connection port on the convergence arm of the Y-connector. Additionally, the multiple lumen tube includes a first lumen and a second lumen that are separated by a wall. The first lumen extends from the proximal end of the multiple lumen tube to the distal end of the multiple lumen tube. The first lumen has an open proximal end and a distal end, the distal end has a plurality of openings. Furthermore, the second lumen has a proximal end and a distal end, the distal end of the second lumen is in fluid communication with the distal end of the first lumen and has a fluid communication opening on the wall between the first lumen and second lumen. The communication opening is adapted to allow air to enter the distal end of the first lumen. The proximal end of the second lumen extends beyond the proximal end of the first lumen, and the proximal end of the second lumen is in fluid communication with the ambient air.

In another embodiment of the present invention, which may be used in combination with any one or more of the preceding aspects or embodiments, there is provided an enteral feeding and suction system which includes a fluid source, a suction assembly, the Y-connector of claim 1, a multiple lumen tube, an administration tubing segment and a suction tubing segment. The suction assembly includes a vacuum and a suction canister. Additionally, the vacuum and the suction canister are connected by a section of vacuum tubing. The multiple lumen tube includes a first lumen and a second lumen. The first lumen has a proximal end and a distal end. The distal end has at least one opening. The proximal end of the first lumen is adapted to attach to the connection port on the convergence arm of the Y-connector. The second lumen has a proximal end and a distal end. The distal end of the second lumen is in fluid communication with the distal end of the first lumen and has an opening on the wall between the first lumen and the second lumen adapted to allow air to enter the distal end of the first lumen. The proximal end of the second lumen extends beyond the proximal end of the first lumen, and the proximal end of the second lumen is in fluid communication with the ambient air and has an enlarged diameter. The administration tubing segment has a first end connected to the fluid source and a second end adapted to connect to the primary arm of the Y-connector. The suction tubing segment has a first end and a second end. The first end of the suction tubing segment is connected to the suction canister, and the second end of the suction tubing segment is adapted to connect to the tapered hollow stem connector on the suction arm.

In another embodiment of the present invention, which may be used in combination with any one or more of the preceding aspects or embodiments, there is provided an enteral feeding and gastric pressure relief system includes a gastric material collection reservoir, a fluid source, the Y-connector of claim 1, a multiple lumen tube, an administration tubing segment, and a relief tubing segment. The gastric material collection reservoir is adapted to collect reflux fluids from the patient's stomach. The multiple lumen tube includes a first lumen and a second lumen. The first lumen has a proximal end and a distal end. The distal end has at least one opening. The proximal end of the first lumen is adapted to attach to the connection port on the convergence arm of the Y-connector. The second lumen has a proximal end and a distal end. The distal end of the second lumen is in fluid communication with the distal end of the first lumen and has an opening on the wall between the first lumen and the second lumen adapted to allow air to enter the distal end of the first lumen. The proximal end of the second lumen extends beyond the proximal end of the first lumen, and the proximal end of the second lumen is in fluid communication with the ambient air and has an enlarged diameter. The administration tubing segment has a first end connected to the fluid source and a second end adapted to connect to the delivery arm of the Y-connector. The relief tubing segment has a first end and a second end. The first end of the relief tubing segment is connected to the gastric material collection reservoir. Additionally, the second end of the relief tubing segment is connected to the open proximal end of the second lumen of the multiple lumen tube.

Additional features and advantages of the disclosed system, method, and apparatus are described in, and will be apparent from, the following Detailed Description and the Figures. The invention is defined in claim 1. Further embodiments are defined in the appended claims. Aspects, embodiments and examples of the present disclosure which do not fall under the scope of the appended claims do not form part of the invention and are merely provided for illustrative purposes.

### BRIEF DESCRIPTION OF THE FIGURES

Fig. 1A is a perspective view of a nasogastric/nasointestinal enteral suction system implementing a dual port tube for suction and feeding, according to an example embodiment of the present disclosure.
Fig. 1B is a perspective view of a nasogastric/nasointestinal enteral feeding system implementing a dual port tube for suction and feeding, according to an example embodiment of the present disclosure.
Fig. 1C is a perspective view of a nasogastric/nasointenstinal enteral feeding and gastric pressure relief system implementing a dual port tube for suction and feeding, according to an example embodiment of the present disclosure.
Fig. 2 is a perspective view of an enteral feeding and gastric pressure relief device, according to an example embodiment of the present disclosure.
Fig. 3A is a side view of a gastric tubing apparatus with an anti-reflux valve removed, according to an example embodiment of the present disclosure.
Fig. 3B is a side view of a gastric tubing apparatus, according to an example embodiment of the present disclosure.
Fig. 3C is an enlarged front view of a multiple lumen tube, according to an example embodiment of the present disclosure.
Fig. 3D is an enlarged cross-sectional view of a multiple lumen tube, according to an example embodiment of the present disclosure.
Fig. 4A is a cross-sectional view of a multiple lumen tube of the gastric tubing system, according to an example embodiment of the present disclosure.
Fig. 4B is a cross-sectional view of a multiple lumen tube of the gastric tubing system, according to an example embodiment of the present disclosure.
Fig. 4C is a cross-sectional view of a multiple lumen tube of the gastric tubing system, according to an example embodiment of the present disclosure.
Fig. 4D is a cross-sectional view of a multiple lumen tube of the gastric tubing system, according to an example embodiment of the present disclosure.
Fig. 5A is a side view of a Y-connector, according to an example embodiment of the present disclosure.
Fig. 5B is a side view of a Y-connector, according to an example embodiment of the present disclosure.
Fig. 5C is an enlarged top view of a securable Y-connector cap.
Fig. 5D is a cross-sectional view of a gastric tubing apparatus with a positioning stylet, according to an example embodiment of the present disclosure.
Fig. 5E is a side view of a Y-connector with a stopcock valve, according to an to an example embodiment of the present disclosure.
Fig. 5F is a side view of a Y-connector with a stopcock valve, according to an example embodiment of the present disclosure.
Fig. 6A is a side view of a Y-connector inner core, according to an example embodiment of the present disclosure.
Fig. 6B is a cross-sectional view of a Y-connector with a material over-layer shown in phantom, according to an example embodiment of the present disclosure.
Fig. 7 is a side view of a Y-connector material over-layer, according to an example embodiment of the present disclosure.
Fig. 8A is a side view of an anti-reflux valve attached to a connector, according to an example embodiment of the present disclosure.
Fig. 8B is an exploded side view of an anti-reflux valve and a connector, according to an example embodiment of the present disclosure.
Figure 9A is a flowchart of an example suction and feeding process using a nasogastric enteral suction system and feeding system.
Figure 9B is a flowchart of example process using an enteral feeding and gastric pressure relief device.

### DETAILED DESCRIPTION OF EXAMPLE EMBODIMENTS

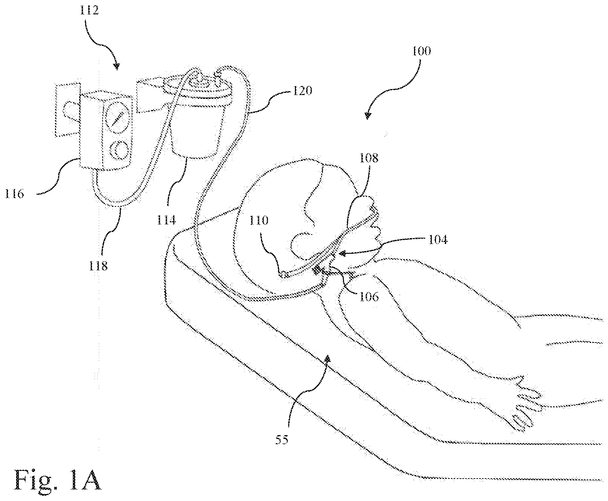

Referring to the Figures, embodiments of an enteral feeding and gastric pressure relief/suction device are disclosed. As seen in an exemplary enteral suction system 100 illustrated in Figure 1A, a gastric tubing apparatus 104 is used in conjunction with a suction assembly 112. The suction assembly 112 may comprise a suction canister 114, a vacuum 116, and a section of vacuum tubing 118. In the illustrated embodiment, the vacuum 116 may be a wall-mounted vacuum or a portable vacuum system. A typical wall-mounted vacuum will produce between 550 and 760 mm Hg (between 73 and 100 kPa) of suction. The vacuum may create a suction pressure in the low pressure range (e.g., 0 to 80 mm Hg (0 to 11 kPa)), medium pressure range (e.g., 80 to 120 mm Hg (11 kPa to 16 kPa), or high pressure range (e.g., greater than 120 mm Hg (16 kPa)). However,it is typically recommended that a patient 55 is exposed to the least amount of vacuum pressure possible for the clinical need, and guidelines state suction pressures typically between 30-40 mm Hg (4.0-5.3 kPa) for nasogastric suctioning (reference Brunner and Suddarth's Textbook of Medical- Surgical Nursing: 12th edition (2009), page 1022). Additionally, intermittent nasogastric suctioning is recommended rather than continuous mode operation of the vacuum system. The wall-mounted vacuum may be controlled with a digital controller or a regulator dial so that the proper suction pressure may be applied through the vacuum tubing 118. The flow rate of gastric material or gastric fluid from the patient's stomach to the suction canister 114 may be accomplished by using the vacuum 116 in combination with the use of suction tubing 120. The term "fluid" as used herein refers to and includes both gaseous and liquid physical states, which may also include solid matter. The suction tubing 120 may be attached to the gastric tubing apparatus 104 during a suction procedure on the patient 55. Once connected, the vacuum 116 may be activated and suction through the suction tube set 120 may be applied at the appropriate suction pressure (e.g., low, medium, or high). Once suction has started, gastric material or gastric fluid enters the gastric tubing apparatus 104 and passes through the suction tubing 120 where it is then separated and collected by the suction canister 114. The gastric tubing apparatus 104 includes a multiple lumen tube 108, a y-connector 106, and may also include an anti-reflux valve 110. The multiple lumen tube 108 may be made from polyurethane, silicone, PVC, or the like. Additionally, the multiple lumen tube 108 may include a vent tube 410 (described in further detail below). Venting helps protect against damage to the gastric mucosa, mucosal tissue tears, bleeding, and increased risk of infections while suction/drainage is occurring as atmospheric air is drawn into the second lumen to equalize the suction vacuum pressure in the stomach once the contents have been emptied. In an example embodiment, the anti-reflux valve 110 is oriented upward to reduce the chance of clogging or wetting of the filter material in the anti-reflux valve 110. This orientation may be achieved by using a clothing clip or other attachment means connected to the multiple lumen tube 108 such that the anti-reflux valve 110 maintains a proper position during treatment. For example, the clothing clip may attach to a pillow or a shirt collar on the patient 55. Other enteral suction arrangements and configurations can be used in connection with the embodiments of the present disclosure illustrated and discussed herein, as will be appreciated by those skilled in the art.

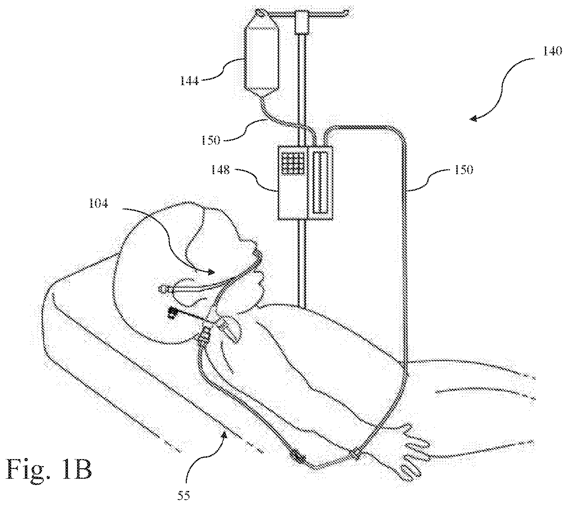

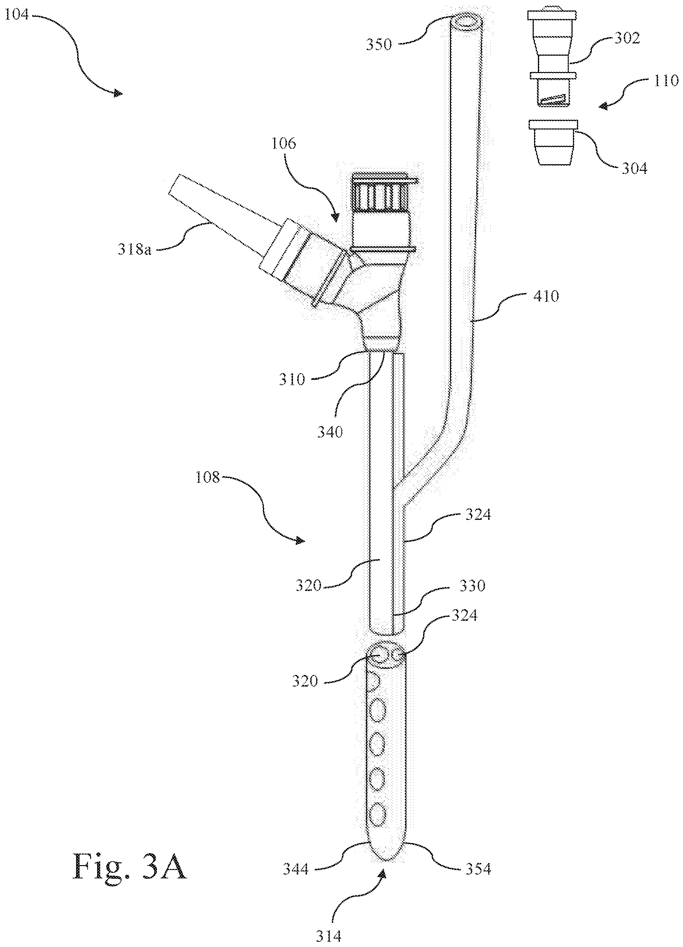

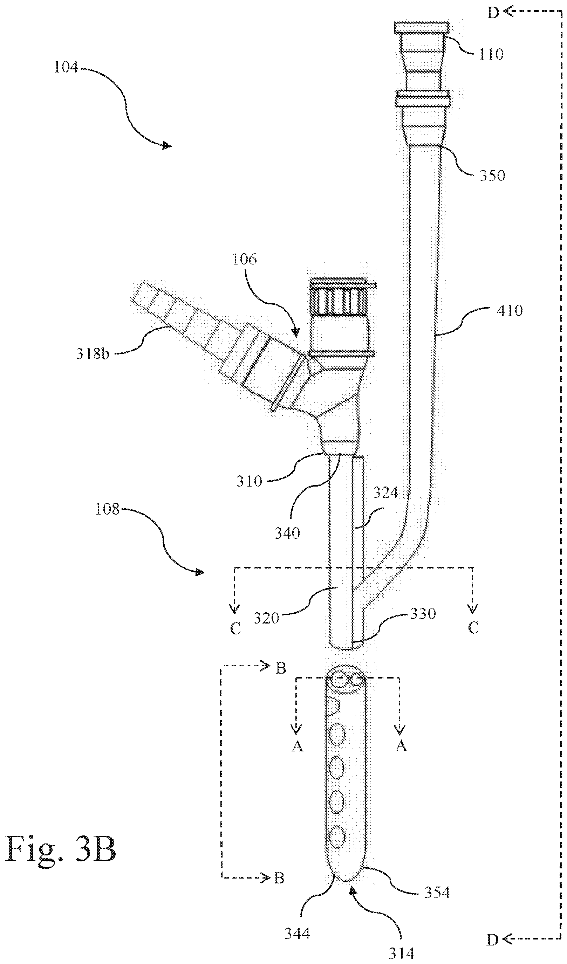

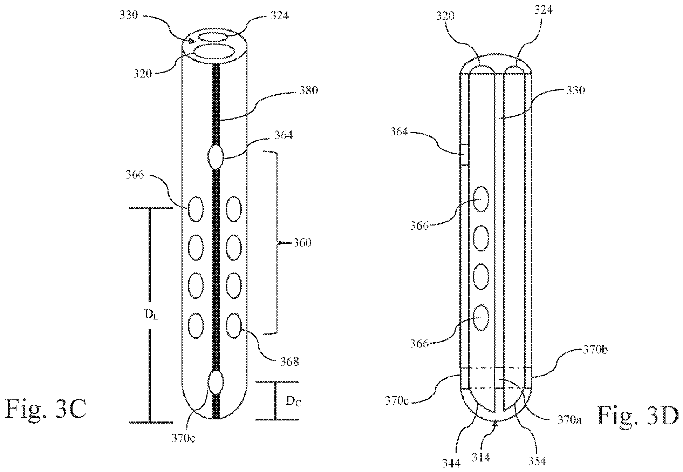

Figure 1B illustrates an example embodiment of an enteral feeding system 140. Nutrient formula is provided from a feeding container 144 (e.g., a suspended feeding bag) and is pumped using pump 148 via administration tubing 150. In an example embodiment, the pump 148 may be a peristaltic pump, however, nutrient formula delivery may also be accomplished without a pump (e.g., using gravity feeding).. The administration tubing 150 may be attached to the gastric tubing apparatus 104 to initiate a feeding procedure on the patient 55. A detail of the multiple lumen suction and feeding connector 104 from Figure 1A and 1B is described in more detail in Figure 3.

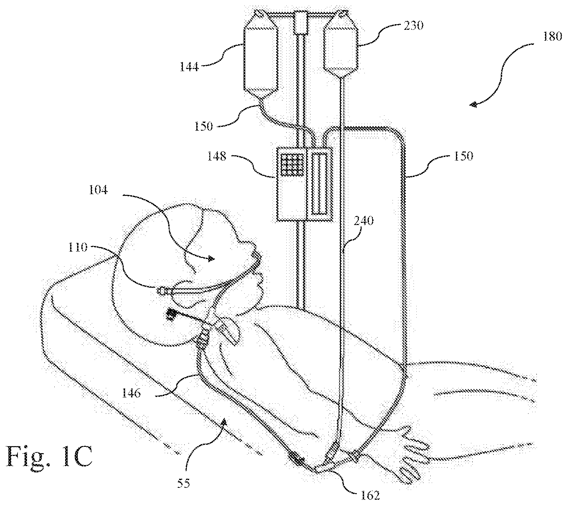

Figure 1C illustrates an example embodiment of an enteral feeding and gastric pressure relief system 180. Nutrient formula is provided from a feeding container 144 (e.g., bag) and is pumped using pump 148 via administration tubing 150. In an example embodiment, the pump 148 may be a peristaltic pump, however, nutrient formula delivery may also be accomplished without a pump (e.g., using gravity feeding). Gastric pressure relief (e.g., gastric decompression) may be provided using a collection reservoir 230 and relief tubing 240 connected to a multi-way connector 162. The relief tubing 240 connects the collection reservoir 230 to a multi-way connector 162. Fluid delivery tubing 146 and administration tubing 150 are also connected to the multi-way connector 162. The fluid delivery tubing 146 may be attached to the gastric tubing apparatus 104 to initiate a feeding procedure on the patient 55. An example embodiment, of the multiple lumen suction and feeding connector 104 from Figure 1A and 1B is described in more detail in Figure 3. In an example embodiment, the anti-reflux valve 110 may be capped or closed to ensure that reflux from the patient passes through the gastric tubing apparatus 104, to the fluid delivery tubing 146 and through the multi-way connector 162 into the relief tubing 240 and into the collection reservoir 230.

Additionally, a one-way check valve or other backflow prevention means can be integrated into the enteral feeding and gastric pressure relief system 180 to prevent retrograde flow back into administration tubing 150 (and thus into feeding container 144). The one-way check valve can be an internal valve in one arm of the multi-way connector 162, or it can be interposed between the multi-way connector 162 and administration tubing 150. The one-way check valve permits flow only in one direction (i.e., toward relief tubing 240 and/or fluid delivery tubing).

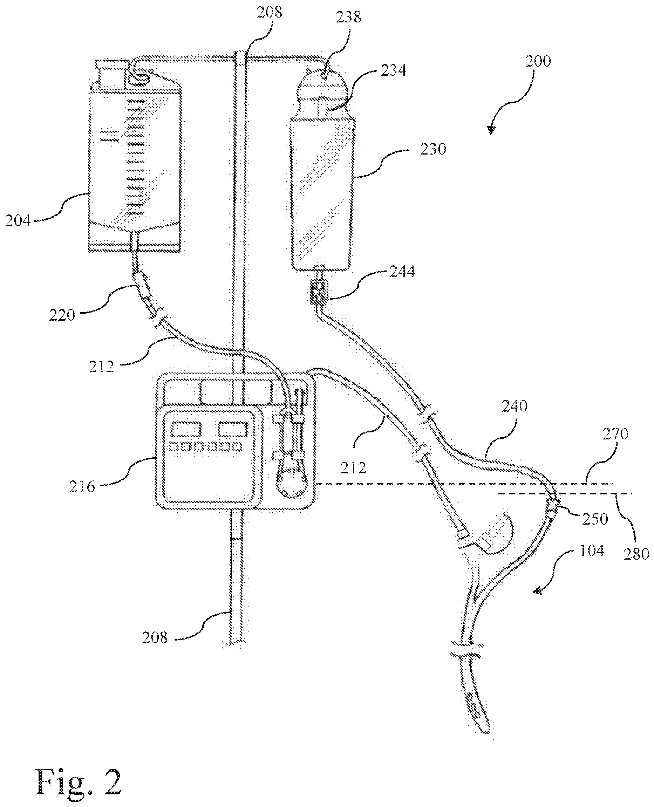

Figure 2 is a perspective view of an enteral feeding and gastric pressure relief device 200. An enteral feeding container 204 containing a selected nutrient formula is suspended from a support stand 208. Other enteral feeding system arrangements and configurations can be used in connection with the embodiments of the present disclosure illustrated and discussed herein, as will be appreciated by those skilled in the art. The flow rate for delivery of nutrient formula from the enteral feeding container 204 through administration tubing 212 may be accomplished through use of an enteral feeding pump 216 in combination with the use of an administration tube set clamping mechanism 220. Nutrient formula flow rate may also be achieved through gravity feed controlled through the combination of a drip chamber and a tubing clamp (e.g., a roller or other adjustable clamp).As explained in greater detail below, gastric pressure relief is achieved through the appropriate connection between the vent lumen of gastric tubing apparatus 104, the relief tubing 240, and the collection reservoir 230. The reflux material collection reservoir 230 is configured to receive materials (i.e., gas and/or liquid) refluxed from a patient's stomach during gastric pressure relief. In Figure 2, the collection reservoir 230 is vented to the ambient atmosphere through a gas vent 234 and is suspended from the support stand 208 by a hanger tab 238. A segment of relief tubing 240 connects the collection reservoir 230 to a gastric tubing apparatus 104; a relief tubing clamping mechanism 244 can be used to selectively control the flow of gases and liquids between the gastric tubing apparatus 104 and the collection reservoir 230. Administration tubing 212 is also connected to the gastric tubing apparatus 104. In an example embodiment, the relief tubing 240 has a connector 250 adapted to connect to an air-vent lumen from the gastric tubing apparatus 104, which will be described further below. For example, if an anti-reflux valve 110 is connected to the air-vent lumen on the gastric tubing apparatus 104, then the anti-reflux valve 110 may be removed and the connector 250 may be attached to the air-vent lumen. In an example embodiment, the connector 250 may be a female mating threaded small-bore connector (e.g., ISO 80369-3).

The relief apparatus as illustrated in Figure 2 comprises a collection reservoir 230, which is vented to ambient atmospheric pressure. To avoid introducing air into the enteral feeding tube through the relief tubing segment and administration tubing segment, the pressure relief apparatus 200 is configured to provide a small column of liquid (e.g., nutrient formula or other liquid) in the relief tubing segment 240 or in the air-vent lumen. This is accomplished by properly positioning the connection between the air-vent lumen of the gastric tubing apparatus 104 and the relief tubing 240 at or slightly below the level of a patient's stomach. For reference and illustrative purposes, the patient's stomach level is indicated in Figure 2 by line 270. For example, if the connection between the air-vent lumen of the gastric tubing apparatus 104 and the relief tubing 240 is at or below that patient stomach level 270 (or, e.g., the patient's mid-axillary line), a small column of liquid (e.g., nutrient formula or other liquid) enters and remains suspended in the relief tubing 240 with the meniscus of the column generally at line 280 in Figure 2 (in close proximity to, or at the same level as the stomach level 270, typically based on the physical condition of the patient). This liquid column prevents air from being drawn into the administration tubing.

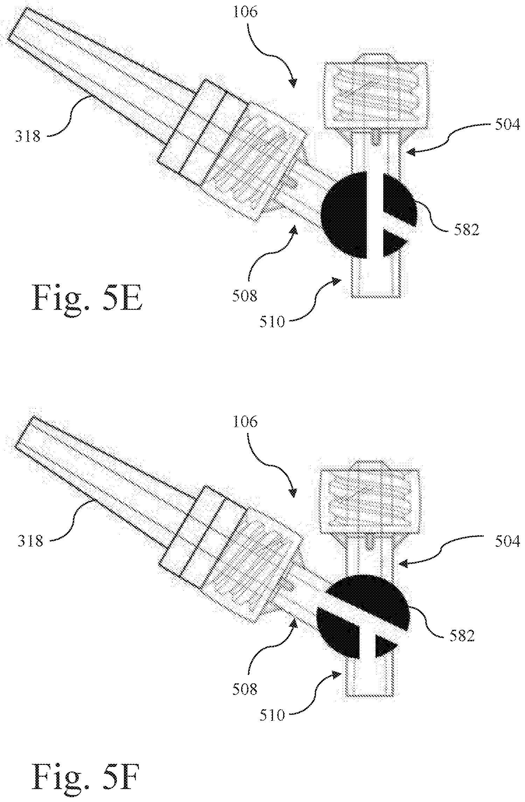

Figures 3A and 3B illustrate side views of a gastric tubing apparatus 104. In Figure 3A, the gastric tubing apparatus 104 is shown with the anti-reflux valve 110 removed. In an example embodiment, the anti-reflux valve 110 may include an anti-reflux filter housing 302, which is adapted to fit into an associated connector 304. For example, connector 304 may be a male enteral only connector (e.g., ISO 80369-3). The gastric tubing apparatus 104 includes a Y-connector 106 coupled to a multiple lumen tube 108 with an open proximal end 310 and a closed distal end 314. The Y-connector 106 also includes a suction connector 318. In an example embodiment, the suction connector 318 may be a constant tapered hollow stem connector 318a, or in another example embodiment, the suction connector 318 may be a stepwise tapered hollow stem connector 318b. The suction connector 318 is tapered, which allows it to frictionally press-fit into the suction tubing 120. In an example embodiment, the suction connector 318 and the remaining structure of the Y-connector may be formed as single, unitary plastic structure. In other embodiments, the suction connector 318 can be affixed to the remaining structure of the Y-connector 106 using adhesive, solvent bonding, radio-frequency (RF) welding and/or any other suitable means of affixing the suction connector 318 to the Y-connector 106. For example, as illustrated in Figures 5E and 5F, the suction connector 318 may be threaded onto the remaining structure of the Y-connector 106. The suction connector 318 may be manufactured of a rigid material such as a suitable copolyester, polyamide, polyethylene, polypropylene, polyacrylonitrile, or the like. An amorphous copolyester, polyamide, polyethylene, polypropylene, polyacrylonitrile, or similar material product typically has suitable appearance, clarity and mold release properties (usable with injection molding, for example). It also provides appropriate rigidity, toughness/durability, hydrolytic stability, heat resistance, and chemical resistance and has been formulated for medical devices.

The multiple lumen tube 108 includes a first lumen 320 and a second lumen 324 separated by a wall 330. The first lumen 320 has a proximal end 340 that is open and a distal end 344 that is closed. The proximal end 340 of the first lumen 320 is coupled to Y-connector 106. The second lumen 324 has a proximal end 350 and a distal end 354. The distal end 354 of the second lumen 324 is in fluid communication with the distal end 354 of the first lumen 320. The proximal end 350 of the second lumen 324 extends beyond the proximal end 340 of the first lumen 320. Additionally, the proximal end 350 of the second lumen 324 is in fluid communication with ambient air and may have an enlarged diameter. The anti-reflux valve 110 is coupled to the proximal end 350 of the second lumen 324.

Figure 3C and Figure 3D show an enlarged view of the multiple lumen tube 108. Figure 3C illustrates an enlarged sectional view along the line B-B in Figure 3B. Figure 3D illustrates an enlarged cross-sectional view along line A-A in Figure 3B. The multiple lumen tube 108 is a flexible hollow tube constructed from a suitable material adapted to maintain flexibility and provide enough structural support to prevent closure or blowout of one of the multiple lumens (320, 324) during suction and/or feeding. Unlike rigid tubes or tubes made from materials that stiffen throughout application, an example embodiment of the multiple lumen tube 108 is produced from a material that maintains flexibility throughout use and advantageously reduces injury to the patient during insertion and removal of the tube. For example, the use of more rigid tubes or tubes that lose flexibility and harden may cause serious problems including necrosis at the nare, damage to the vocal chords, or cause the patient other irritation and discomfort or injury. In an example embodiment, the multiple lumen tube 108 may be made from polyurethane, silicone, or the like, which advantageously provide sufficient flexibility, durability, chemical resistant properties, and ease of molding. In another example embodiment, the multiple lumen tube 108 may be made from polyurethane because PVC may stiffen over time when exposed to the acid environment of the stomach. Instead, polyurethane is "softer" with increasing temperature (e.g., as the temperature increases, the polyurethane becomes more elastic).

As shown in Figure 3C, the multiple lumen tube 108 may also include a radiopaque stripe 380 that extends the length of the tube. In an example embodiment, the radiopaque stripe 380 may be on the outer wall of the first lumen 320. For example, the radiopaque stripe 380 may be a color stripe containing a radiopaque material such as barium sulfate. The radiopaque stripe 380 advantageously enhances the radiopacity of the multiple lumen tube 108 and allows the tube to be visible on x-ray film, which helps a clinician properly confirm location of the tube. In an example embodiment, the radiopaque stripe 380 may cover 25 percent to 50 percent of the outer wall of the first lumen 320. In another example embodiment, the radiopaque stripe 380 may cover 40 percent of the outer wall of the first lumen 320.

The distal end 344 of the first lumen 320 has a plurality of openings 360. The plurality of openings 360 are arranged to provide a gastric material inlet to the first lumen 320 during suction, and provide a nutrient formula outlet of the first lumen 320 during feeding. In an example embodiment, the plurality of openings 360 may include a location hole 364, a first pattern of holes 366, and a second pattern of holes 368. The location hole 364 is placed furthest from the closed distal end 314 of the multiple lumen tube 108 at a predetermined distance (D_{L}) and cuts through the first lumen 320 of the multiple lumen tube 108 to interrupt the radiopaque stripe 380. The interruption of the radiopaque stripe by the location hole 364 advantageously allows the position of the multiple lumen tube 108 to be located using an x-ray to confirm location of this opening. Specifically, the radiopaque stripe 380, the location hole 364, and the distance D_{L} help a clinician determine the position of the closed distal end 314 of the multiple lumen tube 108 because the location hole 364 causes an interruption in the radiopaque stripe 380 and also an interruption in the x-ray visibility. The first pattern of holes 366 and the second pattern of holes 368 may be positioned along each side of the first lumen 320 tube. Additionally, the first pattern of holes 366 may be axially aligned with the second pattern of holes 368. The first pattern of holes 366 and the second pattern of holes 368 are positioned on opposite sides of the first lumen tube to reduce the chance of the plurality of openings 360 from sticking to the stomach wall during the suctioning process, which advantageously reduces the chances of causing a tissue tear, bleeding, gastric mucosal damage, and increased risk of infection. The holes may be oval (as depicted in Figure 3C), circular, or other geometric shapes which are easy to fabricate with the ability to have smooth transitions to the outer tube surface.

In an example embodiment, there may be a fluid communication opening 370 in the wall between the first lumen and the second lumen adapted to allow air to enter the distal end 344 of the first lumen 320 (shown as 370a). The fluid communication opening 370 is placed closest to the closed distal end 314 of the multiple lumen tube 108 at a predetermined distance (D_{C}). Placement of the fluid communication opening 370 closest to the closed distal end 314 of the multiple lumen tube 108 advantageously ensures that ventilation and pressure equalization is provided in the multiple lumen tube 108 at all insertion depths. In an example embodiment, the fluid communication opening 370 may also extend through the outer wall of the second lumen 324 (shown as 370b). Furthermore, in another example embodiment, the fluid communication opening 370 may extend through the outer wall of the second lumen 324, the wall 330 between the first lumen and the second lumen, and the outer wall of the first lumen 320 (shown as 370c). The fluid communication opening 370c may be placed closest to the closed distal end 314 of the multiple lumen tube 108 and may cut through the first lumen 320 of the multiple lumen tube 108 to interrupt the radiopaque stripe 380. The interruption of the radiopaque stripe by the fluid communication opening 370c advantageously allows the position of the most distal fluid communication opening 370c to be located using an x-ray. Location hole 364 and fluid communication opening 370c interrupting the radiopaque stripe may be used independently (e.g., only one hole/opening interrupting the stripe) or together in certain design configurations.

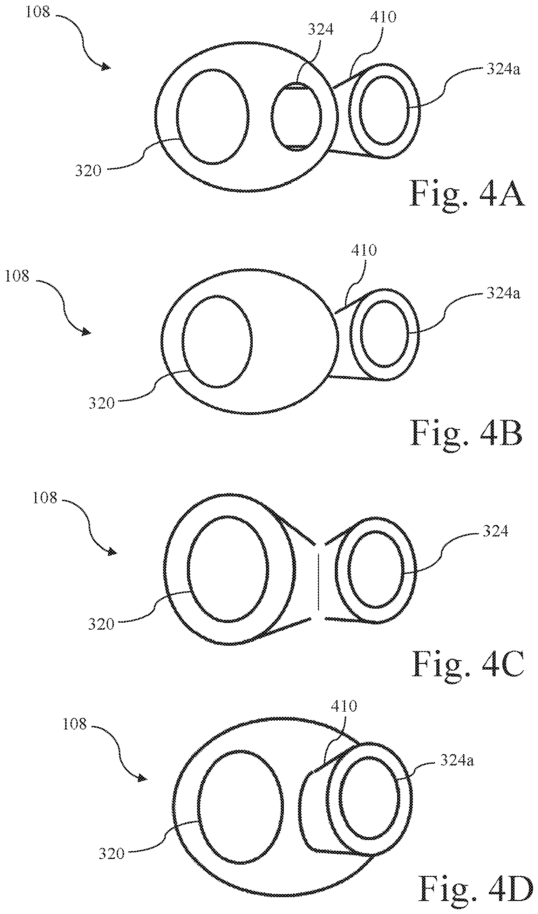

Figures 4A through 4D show cross-sectional views of the multiple lumen tube 108 of the gastric tubing system. The multiple lumen tube 108 may have each of the lumens fully extruded through the proximal end 310 of the multiple lumen tube 108, as shown in Fig 4A. The multiple lumen tube 108 may also include a vent tube 410 that is inserted into second lumen 324 through the outer wall of the multiple lumen tube 108. Venting helps protect against damage to the gastric mucosa, mucosal tissue tears, bleeding, and increased risk of infections while suction/drainage is occurring as atmospheric air is drawn into the second lumen to equalize the suction vacuum pressure in the stomach once the contents have been emptied. In an example embodiment, the vent tube 410 is adapted to block the second lumen 324 passage to the proximal end 310 of the multiple lumen tube 108 and reroute the second lumen 324a passage from the distal end 314 of the multiple lumen tube 108 through the vent tube 410. The vent tube 410 extends out from the side of the second lumen 324 tube of the multiple lumen tube 108 and has a proximal end 350 that extends beyond the proximal end 340 of the first lumen 320, as illustrated in Fig. 3A.

Additionally, as shown in Figure 4B, the multiple lumen tube 108 may consist of a first lumen 320 that has been extruded through the proximal end 310 of the multiple lumen tube 108 and a second lumen 324 extruded to a predetermined height from the proximal end 310 of the multiple lumen tube 108. A vent tube 410 may then couple to the multiple lumen tube 108 at the location where the second lumen 324 extrusion ends to extend the passage of the second lumen 324a from the multiple lumen tube 108 through the vent tube 410.

As shown in Figure 4C, the first lumen 320 and the second lumen 324 of the multiple lumen tube 108 may include two separate lumen tubes that are bonded together. For example, the lumen tubes may be joined together with a biocompatible adhesive, bonded by sonic welding, melted together, or bonded using any other suitable means.

As shown in Figure 4D, the multiple lumen tube 108 may include a first lumen 320 and a second lumen 324a that have been extruded through the proximal end 310 of the multiple lumen tube 108. Additionally, the second lumen 324a may extend through a vent tube 410 that is coupled to the proximal end 310 of the multiple lumen tube 108. For example, the vent tube 410 may be coupled to the multiple lumen tube 108 so that the second lumen 324a extends beyond the proximal end 310 of the multiple lumen tube 108.

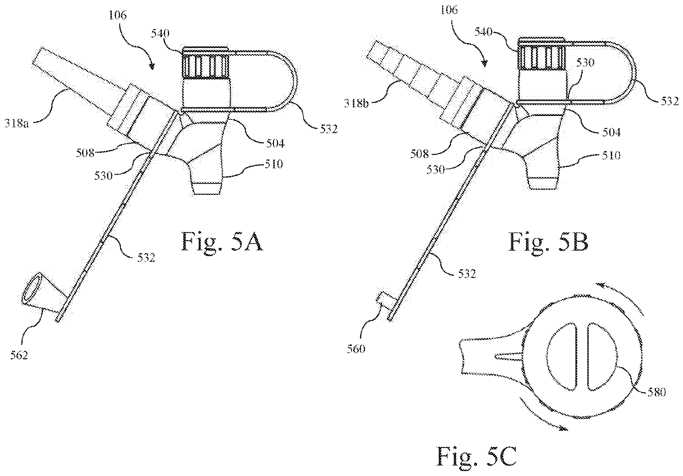

As shown in Figure 5A and 5B, the Y-connector 106 has a delivery arm 504 and a suction arm 508, both of which are connected to a convergence arm 510. It should be appreciated that each arm may also be referred to as a "port". For example, the convergence arm 510 may be referred to as a convergence port or a bidirectional port. The convergence arm 510 has a generally cylindrical stem and is the lower part of the "Y" shape of the Y-connector 106.

In an example embodiment, the delivery arm 504 and convergence arm 510 are generally coaxial, providing a generally linear flow path (of varying diameters in some embodiments) for fluids flowing between the delivery arm 504 and the convergence arm 510. The suction arm 508 is of similar construction to the delivery arm 504, but is oriented at an angle of approximately 60° relative to the delivery arm 504 and convergence arm 510 in the non-limiting exemplary embodiments of the Figures. Other angular orientations between the delivery arm 504 and suction arm 508 may also be implemented. Generally, the likelihood of clogging or other improper flow problems may increase as the angle of the suction arm 508 gets closer to 90°. In some uses, performance may be reduced or otherwise affected with changes in the angular orientation.

The Y-connector 106 may also include a ring connector 530 on each arm. The ring connector 530 or other suitable connector allows a plug or cap to be tethered or otherwise attached to the delivery arm 504. In an example embodiment, the ring connector 530 may include a tether 532 to attach a cap or a plug to each arm. For example, the delivery arm 504 may include a ring connector 530 with tether 532 that is attached to a delivery arm connector cap 540. Additionally, the suction arm 508 may include a ring connector 530 with tether 532 that is attached to a suction connector plug 560 or suction connector cap 562. In an example embodiment, the suction connector cap 562 may also include a plug such that the connector cap 562 seals both the inside and the outside of the suction connector 318.

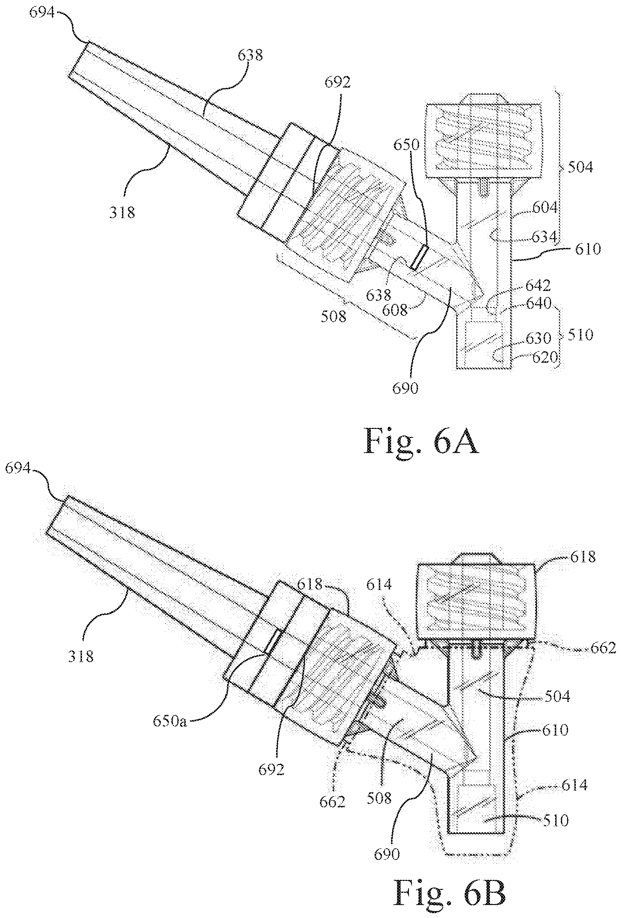

The delivery arm 504 small-bore connector cap 540 is threaded and adapted to engage the mating threaded small-bore connector 618 in Figure 6B. The configuration of the small-bore connector 618 and the small-bore connector cap 540 conform to applicable national and/or international standards (e.g., ISO 80369-3 relative to connectors for enteral applications) for such connectors and the like. Additionally, the delivery arm 504 may be utilized to check properties of the stomach contents (e.g. pH, gastric fluid sampling, etc.) using an enteral syringe. In an example embodiment, the small-bore connector 618 may be configured to be accessible by a syringe or other fluid withdrawal and pumping (e.g., "manmade force-applying") means capable of withdrawing gastric material or reflux material contained in the Y-connector 106 (for example, using an ISO 80369-3 connection configuration). For example, a clinician may fit the end of the small-bore connector 618 with an enteral syringe to perform a pH check of the stomach contents.

The interior of the delivery arm 504 small-bore connector cap 540 may utilize whatever structure is effective to seal an inlet/outlet to prevent ingress of unwanted material into and egress of internal materials from a system incorporating one or more Y-connectors according to the embodiments. The external configuration of the cap enhances ease of use for a nurse, clinician, etc. The cylindrical sidewalls on the periphery of the cap may have gripping means (e.g., knurling, grips, ridges or the like) to provide better gripping of the cap, especially if it is wet or has adhered material that makes disengagement from and/or securement to the Y-connector difficult.

Additionally, the interior of the suction arm suction connector cap may utilize whatever structure is effective to seal the inlet/outlet to prevent ingress of unwanted material into an egress of internal materials from a system. In an example embodiment, a suction connector plug may be used that is adapted to press-fit inside the suction arm channel 638 (as illustrated in Fig. 6A). For example, the suction connector plug 560 may be made of rubber or other suitable compressible material such that it can create an airtight seal at the end of the suction connector 318.

As shown in Figure 5C, the top of the cap may have a double-D configuration 580. This type of multi-indentation cap configuration, which the top of the cap has multiple indentations, recesses, cavities or the like, allows a nurse, clinician, etc. to use a tool (e.g., a cleat or pick) to assist in dislodging a stuck cap (e.g., if a cap was encrusted with material making cap disengagement difficult). Moreover, the multi-indentation cap configuration is advantageous from a fabrication perspective, for example by reducing manufacturing and mold processing challenges.

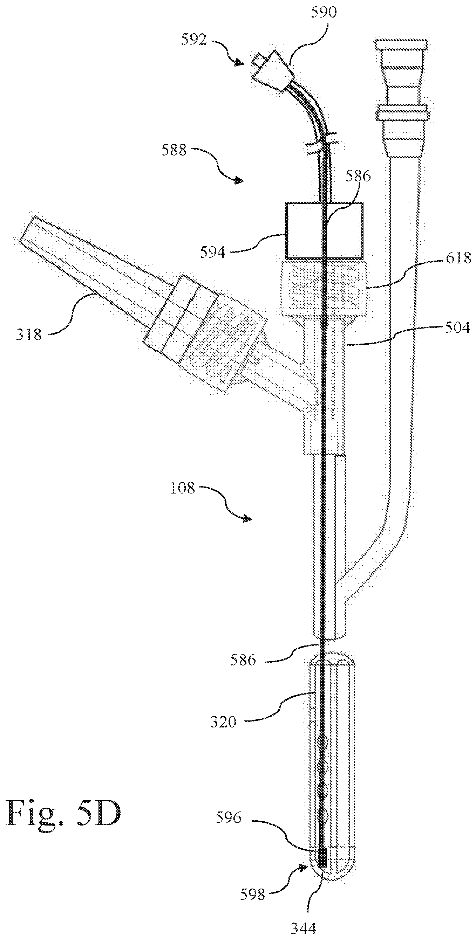

The delivery arm 504 (e.g., feeding arm) may be aligned with the convergence arm 510 to advantageously allow a positioning stylet 586 of a tube assembly guidance system 588 to be used with the gastric tubing apparatus 104. For example, Figure 5D illustrates a cross-sectional view of a gastric tubing apparatus with a positioning stylet along line D-D in Figure 3B. In an example embodiment, the positioning stylet 586 of a tube assembly guidance system 588, such as the CORTRAK® enteral access system, may be used with the delivery arm 504 of the gastric tubing apparatus 104 to assist in positioning the closed distal end 314 of the multiple lumen tube 108 within the patient 55. In an example embodiment, the positioning stylet 586 has an electrical connector 590 included at a first end 592, a union device 594 adapted to couple to the small-bore connector 618 of the delivery arm 504, and a coil 596 included at a second end 598 of the positioning stylet 586. The first end of the positioning stylet 586 may be adapted to couple to the small-bore connector 618 of the delivery arm 504. Additionally, the second end 598 of the positioning stylet 586 may be adapted to move through the first lumen 320 of the multiple lumen tube 108, and the second end 598 may be located within the first lumen 320 and positioned at the distal end 344 of the first lumen 320. For example, the positioning stylet 586 of the tube assembly guidance system 588, such as the CORTRAK® enteral access system, may be preloaded within the multiple lumen tube 108 such that the second end 598 of the positioning stylet 586 is located at the distal end 344 of the first lumen 320. The preloaded placement of the positioning stylet 586 advantageously allows a clinician to determine the placement of the distal end 314 of the multiple lumen tube 108 within the patient.

As shown in Figures 5E and 5F, in another example embodiment, the Y-connector 106 may also incorporate a valve 582 such as a stopcock to selectively control the flow of liquid within the Y-connector 106, or a stopcock type connector may be used to select between channels. Such a connector could be made part of Y-connector 106 with the dual purpose of providing a junction for the administration tubing 150, the suction tubing 120, and the first lumen 320 of the multiple lumen tube 108 and selecting fluid flow in either a path through the suction arm 508 to the suction tubing 120 or through the administration tubing 150 to the delivery arm 504. Using a valve 582, such as a stopcock, may advantageously reduce any chance of improperly using suction tubing 120 or administration tubing 150 connections concurrently because the clinician will have to actively select the fluid flow path. For example, when the valve 582 is positioned in a "delivery arrangement," as illustrated in Fig. 5E, fluid may flow through the delivery arm 504 to the convergence arm 510, while the flow path to the suction arm 508 is blocked. Additionally, when the valve 582 is positioned in a "suction arrangement," as illustrated in Fig. 5F, fluid may flow through convergence arm 510 to the suction arm 508, while the flow path to the delivery arm 504 is blocked.

As shown in Figure 6A and 6B, in an example embodiment, the inner core 610 may be made substantially or completely of a first material, and may be fitted with a material over-layer 614 unit made substantially or completely of a second material. Additionally, the Y-connector may be made from one material, in a single mold, or multiple materials from several molds.

The inner core 610 defines the various arms, channels and connectors of the Y-connector 106. The Y-connector 106 may be made of a rigid material and construction may include a suitable copolyester or the like. An amorphous copolyester product typically has suitable appearance, clarity and mold release properties (usable with injection molding, for example). It also provides appropriate toughness, hydrolytic stability, heat resistance, and chemical resistance and has been formulated for medical devices. This material (and others like it that can be used in Y-connector 106 embodiments claimed herein) provides a strong inner core 610 for the Y-connector 106, including a suitable small-bore connector 618 that meets appropriate standards, guidelines and/or other requirements (e.g., ISO 80369-1:2010 covering small-bore connectors for liquids and gases in healthcare applications). Also, because the rigid material in some Y-connector 106 embodiments is clear, a user is able to confirm visually that connections have been made, to confirm visually that administration is occurring, and to determine visually the cleanliness of the Y-connector 106 before, during and/or after use. The inner core 610 may also be made of a translucent or opaque material, including materials that can possess preselected color characteristics. The inner core 610 may be a single, unitary plastic structure or may be constructed of several components that are combined by adhesive or solvent bonding.

The over-layer 614 may be made of a pliable material such as a thermoplastic elastomer in some embodiments. When such an elastomer is used, it can be a medical-compliant over-mold that adheres to various substrates, including the types of rigid material used in the inner core 610. This type of material has a rubber feel and soft touch and is clear or translucent. Additionally, the material may also be used in injection molding fabrication. The over-layer 614 may also be made of an opaque material, including materials that can possess preselected color characteristics. The over-layer 614 provides a good gripping material for individuals (e.g., nurse, clinician, caregiver, patient, etc.) who are handling and manipulating the Y-connector 106 and also provides a resilient enclosure that permits the use of a rigid material for the inner core 610 while protecting the inner core 610 from breakage, damage, slipperiness and other undesirable characteristics. Moreover, the soft, flexible material over-layer 614 allows the Y-connector 106 to be placed in close proximity to a patient's face or other exposed skin more comfortably. The combination of the inner core 610 and the over-layer 614 permits an organic, smooth shape that allows for ergonomic gripping of the Y-connector 106 during use.

The Y-connector 106 includes a delivery arm 504 and one or more suction arms 508 that provide fluid flow (i.e., gas flow and/or liquid flow) through the Y-connector 106. As seen in in Figure 6A and 6B, the delivery arm 504 includes a generally cylindrical delivery arm stem 604 and a delivery arm channel 634. The suction arm 508 includes a generally cylindrical suction arm stem 608 and a suction arm channel 638. The suction arm stem 608 has a proximal end 690 and a distal end 692. The suction arm channel 638 extends through the suction connector 318. The convergence arm 510 includes a generally cylindrical convergence arm stem 620 and a convergence channel 630 (e.g., connection port, which may also be referred to as a connector port). A suction arm small-bore connector 618 may be positioned at the outer end of the suction arm stem 608. For example, the suction connector 318 may be removable and adapted to screw into the small-bore connector 618. In another example embodiment, the suction connector 318 may be permanently coupled to the suction arm 508. Additional suction and/or delivery arms may be implemented in a similar fashion. The suction arm stem 608 defines an interior suction arm channel 638. In some embodiments, each suction arm stem 608 and its associated small-bore connector 618 are components of an integral inner core 610. In other embodiments, the small-bore connector 618 may be affixed to the suction arm stem 608 using adhesive, solvent bonding, radio-frequency (RF) welding and/or any other suitable means of affixing connector 618 to stem 608.

In an example embodiment, the suction arm channel 638 may include a valve 650, such as a one-way check valve or other backflow prevention means can be integrated into the Y-connector 106 to prevent flow of gastric material during suction to flow back through the suction channel 648 to the convergence arm 510. The valve 650 also advantageously prevents improper materials and/or fluids from being introduced through the suction connector 318 on the suction arm 508. In another example embodiment, the valve 650a may be located within the suction connector 318. The valve 650 may be positioned at any location within the suction arm channel 648 such that it is interposed between the proximal end 690 of the suction arm stem 608 and the outlet end 694 of the hollow stem connector 318. In an example embodiment, the valve 650 may begin to open in a pressure range between 0.1 psi and 1.0 psi (0.7 kPa to 6.9 kPa) (e.g., 5 mm Hg to 50 mm Hg). In another example embodiment, the valve 650 may begin to open around 0.25 psi (1.7 kPa) (e.g., has a crack pressure of 0.25 psi or 13 mm Hg).

The inner bore diameter of the interior channels of the delivery arm 504 and the suction arm 508, respectively, may be different from the inner bore diameter of the convergence arm 510 in some embodiments. The interior channel diameters may be dependent upon the type of connection used between the Y-connector and any tubing segment(s) transmitting fluid to or from the Y-connector. As shown in Figures 6A and 6B, in an example embodiment, an interior annual neck 640 in the interior of the inner core 610 may define a linking channel 642. The interior annular neck 640 defining a linking channel 642 can serve as an anchoring point for a tubing segment, such as a multiple lumen tube, or similar attachments to the convergence arm. In an example embodiment, the linking channel 642, the convergence channel 630 (e.g., connection port), and the delivery arm channel 634 may all have the same diameter. In some embodiments, the tubing segments, such as a multiple lumen tube 108, or similar attachments can be solvent bonded to one or more interior surfaces of the inner core 610 (e.g., to the annular abutment of the neck and/or to the interior surface of the interior channel). Additionally, in an example embodiment, the tubing segments or multiple lumen tube 108 may be removeably press-fit or frictionally fit into the convergence arm. Any suitable means of affixing the Y-connector and the tubing segments may be utilized.

The bores of the delivery arm 504 and suction arm 508 are dictated by dimensions permitted within a given standard or other specific definition (e.g., ISO 80369-3), which can be defined for reducing misconnection potential. The bore size of the convergence arm 510 may be related to the size of the tubing that is attached to it. In an example embodiment, the smallest inner diameter of the convergence arm is no smaller than the inner diameter of the attached tubing. For example, the connection port of the convergence arm 510 may be adapted to receive a multiple lumen tube ranging from size 4 French to size 22 French, although 14 to 18 French sizes are most utilized for nasogastric suctioning applications.

In some Y-connector embodiments usable with enteral feeding systems, for example, the interior channel of the delivery arm (delivery arm channel 634) and the interior channel of the suction arm (suction arm channel 638) may each have an interior bore of approximately 2.90 mm and the inner bore of the interior channel of the convergence arm (convergence channel 630) may be approximately 2.90 mm. In another embodiment, the convergence channel 630 may be larger or may be flared at the end, enabling the convergence channel 630 (e.g., connection port) to fit over larger tubes. In some embodiments, the delivery and suction channels (634, 638) may be connected to the convergence channel 630 using an even smaller diameter linking channel 642. The linking channel 642 may have an inner bore of approximately 2.67 mm and a length of approximately 1.5 mm in some embodiments.

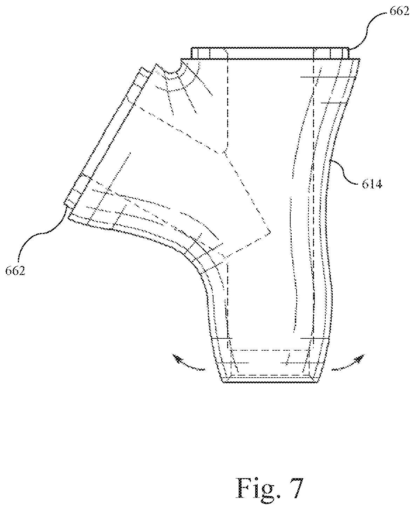

In an example embodiment, a portion of the delivery arm stem 604 may be enclosed in the soft material over-layer 614. As seen in Figure 6A, Figure 6B, and Figure 7, a small circumferential gap 662 is provided between the delivery arm small-bore connector 618 and the portion of the over-layer 614 enclosing the delivery arm stem 604. The gap 662 allows a ring connector 530 or other suitable connector to be attached to the delivery arm 504 so that a small-bore connector cap 540 can be tethered or otherwise attached to the delivery arm 504. The gap 662 enables the ring connector 530 to swing or rotate circumferentially around the small-bore connector 618, thus facilitating movement and positioning of the connector and plug or cap for better access by a clinician or patient to the small-bore connector 618. The gap 662 also ensures that the ring connector 530 remains connected to the Y-connector 106 without the need of a tray, table or other work surface needed to hold the Y-connector ports (e.g., while changing configurations). Additionally, a portion of the suction arm stem 608 is enclosed by an over-layer 614. A small circumferential gap 662 is similarly provided between the suction arm portion of the over-layer and the suction connector 318. The gap 662 allows a ring connector 530 or other suitable connector to be attached to the suction arm 508 so that a suction connector plug 560 or a suction connector cap 562 can be tethered or otherwise attached to the suction arm 508. The gap 662 enables the ring connector to swing or rotate circumferentially around the suction connector 318, thus facilitating movement and positioning of the connector and cap for better access by a clinician or patient to the small-bore connector. The gap 662 also ensures that the ring connector 530 remains with the Y-connector 106.

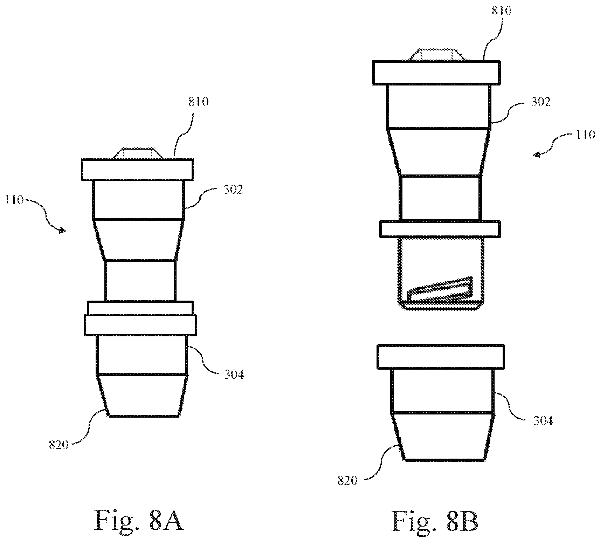

Figure 8A and Figure 8B show an anti-reflux valve 110. The anti-reflux valve 110 includes an anti-reflux filter housing 302 and an associated connector 304. The anti-reflux filter housing 324 includes a membrane or vent material that is adapted to allow airto flow into the top end 810 of the anti-reflux valve 100 while preventing fluid from escaping the second lumen 324. For example, the anti-reflux filter housing 324 may include a one-way valve that only allows air and/or a flushing fluid to be introduced into the second lumen 324 from the anti-reflux valve 110. In an example embodiment, the valve may begin to open in a pressure range between 0.1 psi and 1.0 psi (between 0.7 and 6.9 kPa) (e.g., 5 mm Hg to 50 mm Hg). In another example embodiment, the valve may begin to open around 0.25 psi (1.7 kPa) (e.g., has a crack pressure of 0.25 psi or 13 mm Hg). In another example embodiment, the anti-reflux filter housing may include a membrane or vent material that is adapted to allow air to flow in and out of the top end 810 of the anti-reflux valve 110 to the second lumen 324 and prevent fluid from escaping the second lumen 324. The bottom end 820 of the anti-reflux valve 110 is adapted to couple to the proximal end 350 of the second lumen 324 tube. In an example embodiment, the bottom end 820 of the anti-reflux valve 110 is permanently attached to the multiple lumen tube 108 using an adhesive or any other suitable attachment means. For example, the membrane may include a vent material such as a hydrophobic and/or oliophobic vent materials placed over the sump vent to prevent reflux exit. For example, the vent material may be included in the anti-reflux filter housing 302 as a plug that can be removed from the anti-reflux valve 110 and replaced. Additionally, the anti-reflux valve 110 may include one or more membranes, each membrane may be selected from an appropriate range of materials and configurations (e.g., a hydrophobic and/or oliophobic membrane made of materials such as those produced by Gore and Trinity Technology Group (TTG), for example a membrane material may be an acrylic copolymer membrane cast on a nonwoven nylon support. The membrane material may then be treated for desired performance regarding oliophobicity and/or hydrophobicity.

For example, the breathability (i.e., measure of air permeability typically measured by the Gurley number), liquid surface tension (force exerted by below-the-surface molecules upon those at the surface-air interface), solids surface-free energy (force exerted by below-the-surface molecules upon those at the surface-air interface), and the membrane's wet-out properties (filling the membrane pores with fluid) are important properties for the membrane. In settings where the membrane is likely to come into frequent contact with fluids such as aspirating fluid or fluid nutrient formula, membrane embodiments are used that resist "wetting" that reduces its efficiency.

In an example embodiment, as illustrated in Fig. 8B, the anti-reflux filter housing 302 may be removed from the anti-reflux valve 110 thereby leaving the connector 304 attached to the second lumen 324 of the multiple lumen tube 108. Once the anti-reflux filter housing 302 is removed, it may be replaced, or other tube connections may be inserted into connector 304, such as connector 250 associated with relief tubing 240 to provide gastric relief or an enteral syringe (e.g., an ISO 80369-3 compliant enteral syringe). The anti-reflux filter housing 302 and the connector 304 may be made of polyamide, polyethylene, polypropylene, polyacrylonitrile, copolyester, or the like, which advantageously provide a rigid structure to help prevent misconnections, provides toughness/durability to the components, have chemical resistant properties, and allow for ease of manufacturability.

The top end 810 of the anti-reflux valve 110 may be adapted to attach to a syringe. For example, the top end 810 of the anti-reflux valve 110 may include a male enteral only connector (e.g., ISO 80369-3) that is adapted to receive an enteral syringe (e.g., an ISO 80369-3 compliant enteral syringe). The syringe may be used to force air through the anti-reflux valve 110 to clean the membrane material in a situation where the filter or membrane material becomes clogged with gastric material after wetting occurs. Additionally, the syringe may be used to force reflux material back through the second lumen before wetting occurs. This advantageously prolongs the usable life the anti-reflux valve 110 and continues to maintain a closed system, reducing the instances of opening the system to contamination when an anti-reflux filter housing 302 needs to be replaced. "Open" systems (e.g. one where the patient's gastric aspirate is open to the external environment) also risk clinician and caregiver exposure to the patient's bodily fluids. Such potential exposure to a patient's bodily fluids also require additional Personal Protective Equipment (PPE) per the Centers for Disease Control and Prevention (CDC) and the National Institute for Occupational Safety and Health (NIOSH).

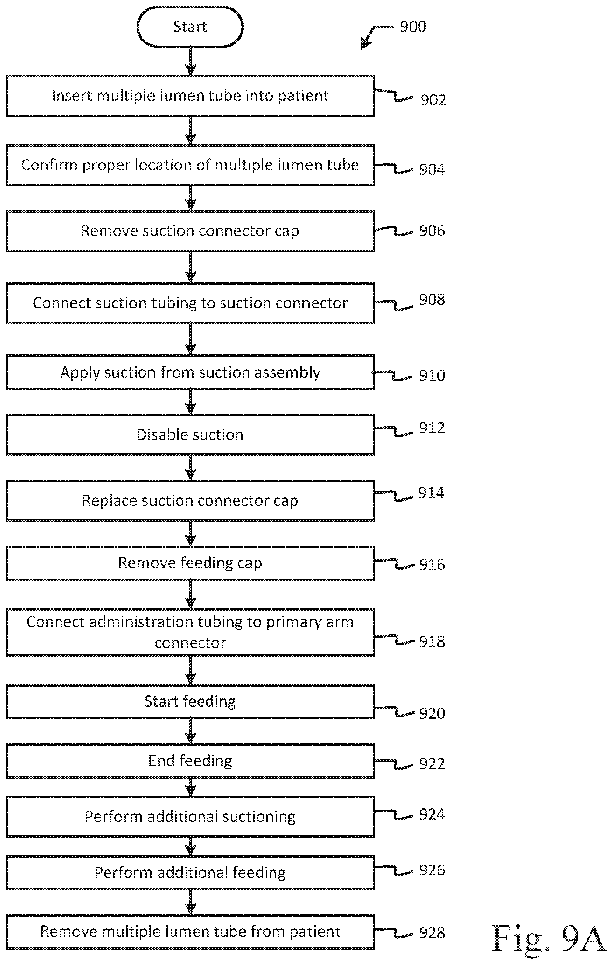

Figure 9A includes a flowchart of an example suction and feeding process 900 using a nasogastric enteral suction system and feeding system. Although process 900 is described with reference to the flowchart illustrated in Fig. 9A, it will be appreciated that many other methods and sequencing of performing the acts associated with the process 900 may be used. For example, the order of many of the blocks may be changed, many blocks may be intermittently repeated or continually performed, certain blocks may be combined with other blacks, and many of the blocks described are optional or may only be contingently performed.

The example process 900 may begin with a clinician inserting the multiple lumen tube 108 from the gastric tubing system 104 into the patient (block 902). Then, the clinician may confirm the proper location of the multiple lumen tube 108. For example, the clinician may remove the delivery arm connector cap 540 and utilize a positioning stylet 586 of a tube assembly guidance system 588 to confirm the proper location of the multiple lumen tube 108 (block 904). For example, the positioning stylet 588 of the tube assembly guidance system, such as the CORTRAK® enteral access system, may be used with the delivery arm 504 of the gastric tubing apparatus 104 to assist in positioning the closed distal end 314 of the multiple lumen tube 108 within the patient 55. Also, for example, the clinician may use x-ray technology and the radiopaque stripe 380 to confirm placement location. The clinician may also obtain a sample of gastric aspirate using an enteral syringe and check pH levels. Once the position of the closed distal end 314 of the multiple lumen tube 108 is confirmed, the clinician may remove the suction connector plug 560 or suction connector cap 562 (block 906). Then, the clinician may connect the suction tubing 120 to the suction connector 318 (block 908) and apply suction from the suction assembly 112 (block 910). The suction assembly 112 may include a vacuum 116 such as a wall-mounted vacuum that can apply different suction pressures based on the patient and the procedure. For example, a neonatal patient may require and/or tolerate a smaller suction pressure than an adult will. When suction is no longer necessary, the clinician may disable the suction by turning the vacuum 116 off (block 912). Then, the clinician may replace the suction connector cap 562 (block 914). If feeding is necessary, the clinician may remove the feeding cap or the delivery arm connector cap 540 (block 916). Additionally, at this point, the clinician may once again confirm the proper location of the multiple lumen tube 108 by utilizing the positioning stylet 586 or by using radiopaque stripe 380. Then, the clinician may connect the administration tubing 212 to the delivery arm 504 via the small-bore connector 618 (block 918). Then, the clinician may start the feeding procedure (block 920). If gravity is being used to induce feeding flow, the clinician may adjust the appropriate administration tube set clamping mechanism 220 to allow fluid such as nutrient formula to flow through the administration tubing 212. If a feeding pump 216 is being used to induce feeding flow, then the clinician may activate the feeding pump at the required setting. Then, the clinician may end the feeding procedure and turn off the feeding pump 216 or close the administration tube set clamping mechanism 220 (block 922). After feeding has been completed or halted (e.g., due to patient discomfort), it may be necessary or beneficial to perform an additional suction procedure. For example, the clinician may perform the necessary steps from blocks 906 through 912 to perform additional suctioning (block 924). After an additional suction procedure, the clinician may initiate an additional feeding procedure by following any of the necessary steps from blocks 914 through 922 (block 926). Finally, the clinician may remove the multiple lumen tube 108 from the patient (block 928).

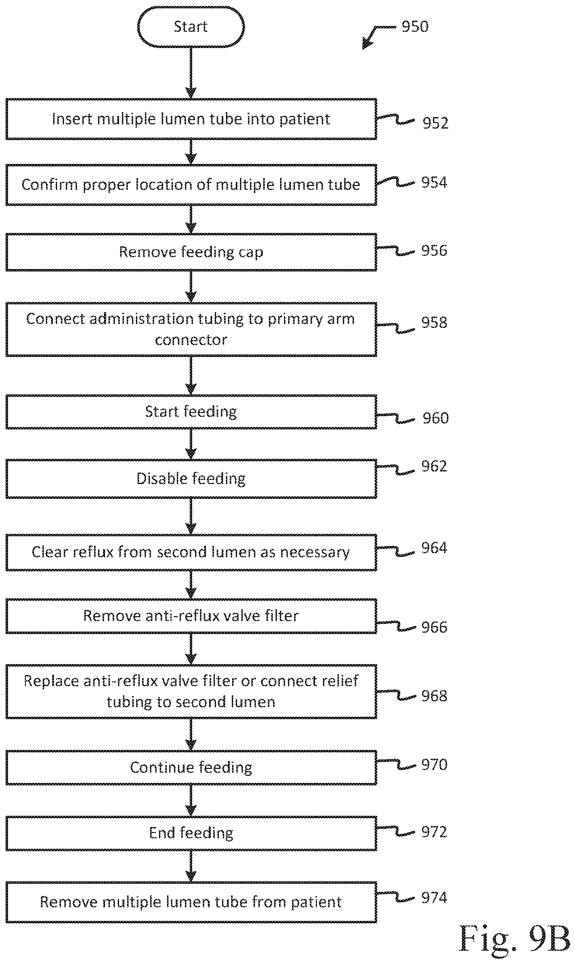

Figure 9B includes a flowchart of example process 950 using an enteral feeding and gastric pressure relief device. Although process 950 is described with reference to the flowchart illustrated in Fig. 9B, it will be appreciated that many other methods of performing the acts associated with the process 950 may be used. For example, the order of many of the blocks may be changed, many blocks may be intermittently repeated or continually performed, certain blocks may be combined with other blacks, and many of the blocks described are optional or may only be contingently performed.

The example process 950 may begin with a clinician inserting the multiple lumen tube 108 from the gastric tubing system 104 into the patient (block 952). Then, the clinician may confirm the proper location of the multiple lumen tube 108. For example, the clinician may remove the delivery arm connector cap 540 and utilize a positioning stylet 586 of a tube assembly guidance system 588 to confirm the proper location of the multiple lumen tube 108 (block 954). For example, the positioning stylet 586 of the tube assembly guidance system 588, such as the CORTRAK® enteral access system, may be used with the delivery arm 504 of the gastric tubing apparatus 104 to assist in positioning the closed distal end 314 of the multiple lumen tube 108 within the patient 55. Also, for example, the clinician may use x-ray technology and the radiopaque stripe 380 to confirm placement location. The clinician may also obtain a sample of gastric aspirate using an enteral syringe and check pH levels. Once the position of the closed distal end 314 of the multiple lumen tube 108 is confirmed, the clinician may remove the feeding cap or the delivery arm connector cap 540 (block 956). Then, the clinician may connect the administration tubing 212 to the suction delivery arm 504 of the Y-connector 106 (block 958) and start feeding from enteral feeding container 204 (block 960). If the patient is experiencing problems with the feeding, and reflux needs to be relieved, the clinician may disable feeding by closing the appropriate administration tube set clamping mechanism 220 or disabling the feeding pump 216 (block 962). The clinician may clear reflux from the second lumen 324 or vent tube 410 as necessary (block 964). For example, the clinician may attach an enteral syringe containing air to the anti-reflux valve 110 to clear reflux from the second lumen 324 or vent tube 410. Then, the clinician may remove anti-reflux filter housing 302 (block 966). Additionally, the clinician may use a syringe containing air to clear reflux from the second lumen 324 or vent tube 410 and/or to remove any debris on the anti-reflux valve membrane or filter before removing the filter housing 302. After the anti-reflux filter housing 302 has been removed, the associated connector 304 of the anti-reflux valve 110 will still be attached to the air-vent lumen of the multiple lumen tube 108. Then, the clinician may replace the filter housing 302 or connect the connector 250 of the relief tubing 240 into the connector 304 that is associated with the anti-reflux valve 110 and is affixed at the proximal end 350 of the second lumen 324 tube (block 968). Then, the clinician may continue feeding while the refluxed materials are collected in the reflux material collection reservoir 230 (block 970). Next, the clinician may end the feeding procedure when a desired amount of nutrient formula has been delivered to the patient 55 (block 972). Finally, the clinician may remove the multiple lumen tube 108 from the patient (block 974).

It should be understood that various changes and modifications to the example embodiments described herein will be apparent to those skilled in the art. Such changes and modifications can be made without departing from the scope of the present subject invention, as set out in the appended claims. It is therefore intended that such changes and modifications be covered by the appended claims.

Aspects of the subject matter described herein may be useful alone or in combination with any one or more of the other aspect described herein. Without limiting the foregoing description, in an exemplary aspect of the present disclosure, a Y-connector for use in a gastric system includes a delivery arm, a suction arm, a one-way valve, and a convergence arm. The suction arm includes a suction arm stem and an outer end. The suction arm stem has a proximal end and a distal end. The suction arm also includes a tapered hollow stem connector adapted to frictionally fit into a section of tubing. The tapered hollow stem connector has a base and an outlet end, and is axially aligned with the suction arm stem. The base of the hollow stem connector is coupled to the outer end of the suction arm, and the tapered hollow stem connector has an outside diameter that decreases from the base of the hollow stem connector to the outlet end of the hollow stem connector. The one- way valve is interposed between the outlet end of the hollow stem connector and the proximal end of the suction arm stem and the valve is adapted to allow fluid to flow in one direction through the suction arm stem. For example, fluid may flow from the base of the hollow stem connector to the outlet end of the hollow stem connector. The delivery arm has a delivery arm stem, and the delivery arm includes a threaded small-bore connector at an outer end of the delivery arm. The convergence arm has a convergence arm stem. Additionally, the convergence arm includes a connection port adapted to receive a multiple lumen tube. The delivery arm stem is in fluid communication with the convergence arm stem, and the suction arm stem is in fluid communication with the convergence arm stem and the delivery arm stem.

In accordance with another exemplary aspect of the present disclosure, which may be used in combination with the preceding aspect, the Y-connector further includes an inner core made of a first material and an over-layer made of a second material. The over-layer encloses at least a portion of the Y-connector body portion.

In accordance with another exemplary aspect of the present disclosure, which may be used in combination with any one or more of the preceding aspects, the over-layer at least partially encloses the inner core.

In accordance with another exemplary aspect of the present disclosure, which may be used in combination with any one or more of the preceding aspects, the first material may be a rigid plastic material.

In accordance with another exemplary aspect of the present disclosure, which may be used in combination with any one or more of the preceding aspects, the second material over-lay may be a pliable material.

In accordance with another exemplary aspect of the present disclosure, which may be used in combination with any one or more of the preceding aspects, the first material may be a transparent copolyester and the second material may be a translucent thermoplastic elastomer.

In accordance with another exemplary aspect of the present disclosure, which may be used in combination with any one or more of the preceding aspects, the outside diameter of the tapered hollow stem connector may decrease in a stepwise fashion.

In accordance with another exemplary aspect of the present disclosure, which may be used in combination with any one or more of the preceding aspects, the outside diameter of the tapered hollow stem connector may decrease in a constant linear fashion.

In accordance with another exemplary aspect of the present disclosure, which may be used in combination with any one or more of the preceding aspects, the connector port of the convergence arm is adapted to receive a multiple lumen tube ranging from size 14 French to size 18 French.

In accordance with another exemplary aspect of the present disclosure, which may be used in combination with any one or more of the preceding aspects, the Y-connector further includes a first cap configured to seal the delivery arm and a second cap configured to seal the tapered hollow stem connector on the suction arm.

In accordance with another exemplary aspect of the present disclosure, which may be used in combination with any one or more of the preceding aspects, the first cap may be coupled to the delivery arm with a first tether configured to circumferentially rotate the first cap about the delivery arm. Additionally, the second cap may be coupled to the suction arm with a second tether configured to circumferentially rotate the second cap about the suction arm.

In accordance with another exemplary aspect of the present disclosure, which may be used in combination with any one or more of the preceding aspects, the second cap is adapted to fit over the entire surface of the tapered hollow stem connector.

In accordance with another exemplary aspect of the present disclosure, which may be used in combination with any one or more of the preceding aspects, the second cap is adapted to fit over an end portion of the tapered hollow stem connector.

In accordance with another exemplary aspect of the present disclosure, which may be used in combination with any one or more of the preceding aspects, the second cap includes a plug adapted to frictionally fit inside the hollow portion of the outer end of the tapered hollow stem connector.

In accordance with another exemplary aspect of the present disclosure, which may be used in combination with any one or more of the preceding aspects, a gastric tubing system includes a Y-connector and a multiple lumen tube. The Y-connector includes a delivery arm, a suction arm, a one-way valve, and a convergence arm. The suction arm includes a suction arm stem and an outer end. The suction arm stem has a proximal end and a distal end. The suction arm also includes a tapered hollow stem connector adapted to frictionally fit into a section of tubing. The tapered hollow stem connector has a base and an outlet end, and is axially aligned with the suction arm stem. The base of the hollow stem connector is coupled to the outer end of the suction arm, and the tapered hollow stem connector has an outside diameter that decreases from the base of the hollow stem connector to the outlet end of the hollow stem connector. The one-way valve is interposed between the outlet end of the hollow stem connector and the proximal end of the suction arm stem, and the valve is adapted to allow fluid to flow in one direction through the suction arm stem. For example, fluid may flow from the base of the hollow stem connector to the outlet end of the hollow stem connector. The delivery arm has a delivery arm stem, and the delivery arm includes a threaded small-bore connector at an outer end of the delivery arm. The convergence arm has a convergence arm stem. Additionally, the convergence arm includes a connection port adapted to receive a multiple lumen tube. The delivery arm stem is in fluid communication with the convergence arm stem, and the suction arm stem is in fluid communication with the convergence arm stem and the delivery arm stem. The multiple lumen tube has an open proximal end and a closed distal end. The open proximal end of the multiple lumen tube is adapted to couple to the connection port on the convergence arm of the Y-connector. Additionally, the multiple lumen tube includes a first lumen and a second lumen that are separated by a wall. The first lumen extends from the proximal end of the multiple lumen tube to the distal end of the multiple lumen tube. The first lumen has an open proximal end and a distal end, the distal end has a plurality of openings. Furthermore, the second lumen has a proximal end and a distal end, the distal end of the second lumen is in fluid communication with the distal end of the first lumen and has a fluid communication opening on the wall between the first lumen and second lumen. The communication opening is adapted to allow air to enter the distal end of the first lumen. The proximal end of the second lumen extends beyond the proximal end of the first lumen, and the proximal end of the second lumen is in fluid communication with the ambient air.

In accordance with another exemplary aspect of the present disclosure, which may be used in combination with the preceding aspect, the proximal end of the second lumen has an enlarged diameter.

In accordance with another exemplary aspect of the present disclosure, which may be used in combination with any one or more of the preceding aspects, the gastric tubing system further includes a positioning stylet. The positioning stylet has a first end and a second end, and also includes a union device. The union device is adapted to couple to the small-bore connector of the delivery arm of the Y-connector. The first end of the positioning stylet includes an electrical connector. The second end of the positioning stylet includes a coil and is adapted to move through the first lumen of the multiple lumen tube. Additionally, the second end of the positioning stylet is located within the first lumen of the multiple lumen tube and is positioned at the distal end of the first lumen.

In accordance with another exemplary aspect of the present disclosure, which may be used in combination with any one or more of the preceding aspects, the gastric tubing system further includes an anti-reflux valve attached to the proximal end of the second lumen.

In accordance with another exemplary aspect of the present disclosure, which may be used in combination with any one or more of the preceding aspects, the anti-reflux valve has a top end and a bottom and includes a one-way valve adapted to allow fluid to flow in one direction through the anti-reflux valve from the top end of the anti-reflux valve to the second lumen.

In accordance with another exemplary aspect of the present disclosure, which may be used in combination with any one or more of the preceding aspects, the anti-reflux valve has a top end and a bottom and includes an anti-reflux housing and a connector. The housing has a membrane that is adapted to obstruct fluid flow while allowing air to flow in both directions. The connector is adapted to receive the anti-reflux housing.

In accordance with another exemplary aspect of the present disclosure, which may be used in combination with any one or more of the preceding aspects, the top end of the anti-reflux valve is adapted to couple to a syringe.

In accordance with another exemplary aspect of the present disclosure, which may be used in combination with any one or more of the preceding aspects, an enteral feeding and suction system includes a fluid source, a suction assembly, a multiple lumen tube, an administration tubing segment and a suction tubing segment. The suction assembly includes a vacuum and a suction canister. Additionally, the vacuum and the suction canister are connected by a section of vacuum tubing. The Y-connector includes a delivery arm, a suction arm, a one-way valve, and a convergence arm. The suction arm includes a suction arm stem and an outer end. The suction arm stem has a proximal end and a distal end. The suction arm also includes a tapered hollow stem connector adapted to frictionally fit into a section of tubing. The tapered hollow stem connector has a base and an outlet end, and is axially aligned with the suction arm stem. The base of the hollow stem connector is coupled to the outer end of the suction arm, and the tapered hollow stem connector has an outside diameter that decreases from the base of the hollow stem connector to the outlet end of the hollow stem connector. The one-way valve is interposed between the outlet end of the hollow stem connector and the proximal end of the suction arm stem and the valve is adapted to allow fluid to flow in one direction through the suction arm stem. For example, fluid may flow from the base of the hollow stem connector to the outlet end of the hollow stem connector. The delivery arm has a delivery arm stem, and the delivery arm includes a threaded small-bore connector at an outer end of the delivery arm. The convergence arm has a convergence arm stem. Additionally, the convergence arm includes a connection port adapted to receive a multiple lumen tube. The delivery arm stem is in fluid communication with the convergence arm stem, and the suction arm stem is in fluid communication with the convergence arm stem and the delivery arm stem. The multiple lumen tube has a proximal end and a distal end. The multiple lumen tube includes a first lumen and a second lumen. The first lumen has a proximal end and a distal end. The distal end has at least one opening. The proximal end of the first lumen is adapted to attach to the connection port on the convergence arm of the Y-connector. The second lumen has a proximal end and a distal end. The distal end of the second lumen is in fluid communication with the distal end of the first lumen and has an opening on the wall between the first lumen and the second lumen adapted to allow air to enter the distal end of the first lumen. The proximal end of the second lumen extends beyond the proximal end of the first lumen, and the proximal end of the second lumen is in fluid communication with the ambient air and has an enlarged diameter. The administration tubing segment has a first end connected to the fluid source and a second end adapted to connect to the primary arm of the Y-connector. The suction tubing segment has a first end and a second end. The first end of the suction tubing segment is connected to the suction canister, and the second end of the suction tubing segment is adapted to connect to the tapered hollow stem connector on the secondary arm.

In accordance with another exemplary aspect of the present disclosure, which may be used in combination with any one or more of the preceding aspect, the enteral feeding and suction system further includes an enteral feeding pump.

In accordance with another exemplary aspect of the present disclosure, which may be used in combination with any one or more of the preceding aspects, the enteral feeding and suction system further includes an anti-reflux valve attached to the proximal end of the second lumen.

In accordance with another exemplary aspect of the present disclosure, which may be used in combination with any one or more of the preceding aspects, an enteral feeding and gastric pressure relief system includes a gastric material collection reservoir, a fluid source, a Y-connector, a multiple lumen tube, an administration tubing segment, and a relief tubing segment. The gastric material collection reservoir is adapted to collect reflux fluids from the patient's stomach. The Y-connector includes a delivery arm, a suction arm, a one-way valve, and a convergence arm. The suction arm includes a suction arm stem and an outer end. The suction arm stem has a proximal end and a distal end. The suction arm also includes a tapered hollow stem connector adapted to frictionally fit into a section of tubing. The tapered hollow stem connector has a base and an outlet end, and is axially aligned with the suction arm stem. The base of the hollow stem connector is coupled to the outer end of the suction arm, and the tapered hollow stem connector has an outside diameter that decreases from the base of the hollow stem connector to the outlet end of the hollow stem connector. The one-way valve is interposed between the outlet end of the hollow stem connector and the proximal end of the suction arm stem and the valve is adapted to allow fluid to flow in one direction through the suction arm stem. For example, fluid may flow from the base of the hollow stem connector to the outlet end of the hollow stem connector. The delivery arm has a delivery arm stem, and the delivery arm includes a threaded small-bore connector at an outer end of the delivery arm. The convergence arm has a convergence arm stem. Additionally, the convergence arm includes a connection port adapted to receive a multiple lumen tube. The delivery arm stem is in fluid communication with the convergence arm stem, and the suction arm stem is in fluid communication with the convergence arm stem and the delivery arm stem. The multiple lumen tube includes a first lumen and a second lumen. The first lumen has a proximal end and a distal end. The distal end has at least one opening. The proximal end of the first lumen is adapted to attach to the connection port on the convergence arm of the Y-connector. The second lumen has a proximal end and a distal end. The distal end of the second lumen is in fluid communication with the distal end of the first lumen and has an opening on the wall between the first lumen and the second lumen adapted to allow air to enter the distal end of the first lumen. The proximal end of the second lumen extends beyond the proximal end of the first lumen, and the proximal end of the second lumen is in fluid communication with the ambient air and has an enlarged diameter. The administration tubing segment has a first end connected to the fluid source and a second end adapted to connect to the delivery arm of the Y-connector. The relief tubing segment has a first end and a second end. The first end of the relief tubing segment is connected to the gastric material collection reservoir. Additionally, the second end of the relief tubing segment is connected to the open proximal end of the second lumen of the multiple lumen tube.

In accordance with another exemplary aspect of the present disclosure, which may be used in combination with the preceding aspect, the system further includes an anti-reflux valve connector attached to the proximal end of the second lumen.

The many features and advantages of the present disclosure are apparent from the written description, and thus, the appended claims are intended to cover all such features and advantages of the disclosure. Further, since numerous modifications and changes will readily occur to those skilled in the art, the present disclosure is not limited to the exact construction and operation as illustrated and described. Therefore, the described embodiments should be taken as illustrative and not restrictive, and the disclosure should not be limited to the details given herein but should be defined by the following claims.

## Claims

1. A Y-connector (106) for use in a gastric system (104) comprising:
a suction arm (508) having a suction arm stem (608) and an outer end, wherein
the suction arm stem (608) has a proximal end (690) and a distal end (692), the suction arm (508) includes a tapered hollow stem connector (318a,b) adapted to frictionally fit into a section of tubing (120),
the tapered hollow stem connector (318a,b) has a base and an outlet end (694),
the tapered hollow stem connector (318a,b) is axially aligned with the suction arm stem (608) and the base of the hollow stem connector (318a,b) is coupled to the outer end of the suction arm (508), and
the tapered hollow stem connector (318a,b) has an outside diameter that decreases from the base of the hollow stem connector (318a,b) to the outlet end (694) of the hollow stem connector (318a,b);
a one-way valve (650, 650a) interposed between the outlet end (694) of the hollow stem connector (318a,b) and the proximal end (690) of the suction arm stem (608) adapted to allow fluid to flow in one direction through the suction arm stem (608) from the base of the hollow stem connector (318a,b) to the outlet end (694) of the hollow stem connector (318a,b);
a delivery arm (504) having a delivery arm stem (604), wherein the delivery arm (504) includes a threaded small-bore connector (618) at an outer end of the delivery arm (504); and
a convergence arm (510) having a convergence arm stem (620), wherein
the convergence arm (510) includes a connection port (630) adapted to receive a multiple lumen tube (108),
the delivery arm stem (604) is in fluid communication with the convergence arm stem (620), and
the suction arm stem (608) is in fluid communication with the convergence arm stem (620) and the delivery arm stem (604), wherein an interior annular neck (640) defines a linking channel (642) in the convergence arm (510).

2. The Y-connector (106) of Claim 1, wherein the Y-connector (106) includes an inner core (610) made of a first material and an over- layer (614) made of a second material, the over-layer (614) enclosing at least a portion of the Y-connector body portion.

3. The Y-connector (106) of Claim 2, wherein the over-layer (614) at least partially encloses the inner core (610); and/or
wherein the first material is a rigid plastic material; and/or
wherein the second material over-layer (614) is made from a pliable material; and/or
wherein the first material is a transparent copolyester and further wherein the second material is a translucent thermoplastic elastomer.

4. The Y-connector (106) of Claim 1, wherein the outside diameter of the tapered hollow stem connector (318a,b) decreases in a stepwise fashion; or
wherein the outside diameter of the tapered hollow stem connector (318a,b) decreases in a constant linear fashion.

5. The Y-connector (106) of Claim 1 , wherein the connection port (630) of the convergence arm (510) is adapted to receive a multiple lumen tube (108) ranging from size 14 French to size 18 French.

6. The Y-connector (106) of Claim 1, further comprising a first cap (540) configured to seal the delivery arm (504) and a second cap (562) configured to seal the tapered hollow stem connector (318a,b) on the suction arm (508);
preferably wherein the first cap (540) is coupled to the delivery arm (504) with a first tether (532) configured to circumferentially rotate the first cap (540) about the delivery arm (504) and the second cap (562) is coupled to the suction arm (508) with a second tether (532) configured to circumferentially rotate the second cap (562) about the suction arm (508).

7. The Y-connector (106) of Claim 6, wherein the second cap (562) is adapted to fit over the entire surface of the tapered hollow stem connector (318a,b); or wherein the second cap (562) is adapted to fit over an end portion of the tapered hollow stem connector (318a,b); or
wherein the second cap (562) includes a plug (560) adapted to frictionally fit inside the hollow portion of the outer end of the tapered hollow stem connector (318a,b).

8. A gastric tubing system (104) comprising:
the Y-connector (106) of claim 1; and
a multiple lumen tube (108) having an open proximal end (310) and a closed distal end (314), wherein
the open proximal end (310) of the multiple lumen tube (108) is adapted to couple to the connection port (630) on the convergence arm (510) of the Y-connector (106),
the multiple lumen tube (108) includes a first lumen (320) and a second lumen (324) that are separated by a wall (330),
the first lumen (320) extends from the proximal end (310) of the multiple lumen tube (108) to the distal end (314) of the multiple lumen tube (108),
the first lumen (320) has an open proximal end (340) and a distal end (344), the distal end (344) has a plurality of openings (360),
the second lumen (324) has a proximal end (350) and a distal end (354), the distal end (354) of the second lumen (324) is in fluid communication with the distal end (344) of the first lumen (320) and has a fluid communication opening (370a) on the wall (330) between the first lumen (320) and second lumen (324) adapted to allow air to enter the distal end (344) of the first lumen (320),
the proximal end (350) of the second lumen (324) extends beyond the proximal end (340) of the first lumen (320), and
the proximal end (350) of the second lumen (324) is in fluid communication with the ambient air.

9. The gastric tubing system (104) of Claim 8, wherein the proximal end (350) of the second lumen (324) has an enlarged diameter; and/or
further comprising a positioning stylet (586) having a first end (592) and a second end (598), wherein
the positioning stylet (586) includes a union device (594) adapted to couple to the small-bore connector (618) of the delivery arm (504),
the first end (592) of the positing stylet (586) includes an electrical connector (590),
the second end (598) of the positioning stylet (586) includes a coil (596) that is adapted to move through the first lumen (320) of the multiple lumen tube (108), and
the second end (598) of the positioning stylet (586) is located within the first lumen (320) of the multiple lumen tube (108) at the distal end (344) of the first lumen (320).

10. The gastric tubing system of Claim 8, further comprising an anti-reflux valve (110) attached to the proximal end (350) of the second lumen (324).

11. The gastric tubing system of Claim 10, wherein the anti-reflux valve (110) has a top end (810) and a bottom end (820), the anti-reflux valve (110) including:
a one-way valve adapted to allow fluid to flow in one direction through the anti-reflux valve (110) from the top end (810) of the anti-reflux valve (110) to the second lumen (324); or
the anti-reflux valve (110) including:
an anti-reflux filter housing (302) having a membrane; and
a connector (304), wherein
the connector (304) is adapted to receive the anti-reflux filter housing (302), and
the membrane is adapted to obstruct fluid flow while allowing air to flow in both directions.

12. The system of Claim 11, wherein the top end (810) of the anti-reflux valve (110) is adapted to couple to a syringe.

13. An enteral feeding and suction system comprising:
a fluid source (144, 204);
a suction assembly (112) having a vacuum (116) and a suction canister (114), wherein the vacuum (116) and the suction canister (114) are connected by a section of vacuum tubing (118);
the Y-connector (106) of claim 1;
a multiple lumen tube (108) having a proximal end (310) and a distal end (314), wherein
the multiple lumen tube (108) includes a first lumen (320) and a second lumen (324),
the first lumen (320) has a proximal end (340) and a distal end (344), the distal end (344) has at least one opening (360),
the proximal end (340) of the first lumen (320) adapted to attach to the connection port (630) on the convergence arm (510) of the Y-connector (106),
the second lumen (324) having a proximal end (350) and a distal end (354), the distal end (354) of the second lumen (324) in fluid communication with the distal end (344) of the first lumen (320) and having an opening (370a) on a wall (330) between the first lumen (320) and second lumen (324) adapted to allow air to enter the distal end (344) of the first lumen (320),
the proximal end (350) of the second lumen (324) extending beyond the proximal end (340) of the first lumen (320), and
the proximal end (350) of the second lumen (324) open to ambient air and having an enlarged diameter;
an administration tubing segment (150, 212), wherein the administration tubing segment (150, 212) has a first end connected to the fluid source (144, 204) and a second end adapted to connect to the delivery arm (504) of the Y-connector (106); and
a suction tubing segment (120), wherein
the suction tubing segment (120) has a first end and a second end,
the first end of the suction tubing segment (120) is connected to the suction canister (114), and
the second end of the suction tubing segment (120) is adapted to connect to the tapered hollow stem connector (318a,b) on the suction arm (508).

14. The system of Claim 13, further comprising an enteral feeding pump (216); and/or
further comprising an anti-reflux valve (110) attached to the proximal end (350) of the second lumen (324).

15. An enteral feeding and gastric pressure relief system comprising:
a gastric material collection reservoir (230), wherein the gastric material collection reservoir (230) is adapted to collect reflux fluids from a patient's stomach;
a fluid source (144, 204);
the Y-connector (106) of claim 1;
a multiple lumen tube (108) having a proximal end (310) and a distal end (314), wherein
the multiple lumen tube (108) includes a first lumen (320) and a second lumen (324),
the first lumen (320) has a proximal end (340) and a distal end (344), the distal end (344) has at least one opening (360),
the proximal end of the first lumen (320) adapted to attach to the connection port (630) on the convergence arm (510) of the Y-connector (106),
the second lumen (324) having a proximal end (350) and a distal end (354), the distal end (354) of the second lumen (324) in fluid communication with the distal end (344) of the first lumen (320) and having an opening (370a) on a wall (330) between the first lumen (320) and second lumen (324) adapted to allow air to enter the distal end (344) of the first lumen (320),
the proximal end (350) of the second lumen (324) extending beyond the proximal end (340) of the first lumen (320), and
the proximal end (350) of the second lumen (324) open to ambient air and having an enlarged diameter;
an administration tubing segment (150, 212), wherein the administration tubing segment (150, 212) has a first end connected to the fluid source (144, 204) and a second end adapted to connect to the suction arm (508) of the Y-connector (106); and
a relief tubing segment (240), wherein
the relief tubing segment (240) has a first end and a second end, and the first end of the relief tubing segment (240) is connected to the gastric material collection reservoir (230), and the second end of the relief tubing segment (240) is connected to the open proximal end (350) of the second lumen (324) of the multiple lumen tube (108); and
preferably further comprising an anti-reflux valve connector (304) attached to the proximal end (350) of the second lumen (324).

## Patentansprüche

1. Y-Steckverbinder (106) zur Verwendung in einem Magen-System (104), umfassend:
einen Saugarm (508), der einen Saugarmschaft (608) und ein äußeres Ende aufweist, wobei
der Saugarmschaft (608) ein proximales Ende (690) und ein distales Ende (692) aufweist,
der Saugarm (508) einen sich verjüngenden Hohlschaftsteckverbinder (318a, b) beinhaltet, der angepasst ist, um reibschlüssig in einem Schlauchabschnitt (120) zu sein,
wobei der sich verjüngende Hohlschaftsteckverbinder (318a, b) eine Basis und ein Auslassende (694) aufweist,
der sich verjüngende Hohlschaftsteckverbinder (318a, b) axial mit dem Saugarmschaft (608) ausgerichtet ist und die Basis des Hohlschaftsteckverbinders (318a, b) mit dem äußeren Ende des Saugarmes (508) gekoppelt ist, und
der sich verjüngende Hohlschaftsteckverbinder (318a, b) einen Außendurchmesser aufweist, der von der Basis des Hohlschaftsteckverbinders (318a, b) zu dem Auslassende (694) des Hohlschaftsteckverbinders (318a, b) abnimmt;
ein Einwegventil (650, 650a), das zwischen dem Auslassende (694) des Hohlschaftsteckverbinders (318a, b) und dem proximalen Ende (690) des Saugarmschafts (608) eingesetzt ist, der angepasst ist, um es Fluid zu erlauben, in einer Richtung durch den Saugarmschaft (608) von der Basis des Hohlschaftsteckverbinders (318a, b) zu dem Auslassende (694) des Hohlschaftsteckverbinders (318a, b) zu fließen;
einen Abgabearm (504), der einen Abgabearmschaft (604) aufweist, wobei der Abgabearm (504) einen Gewinde-Steckverbinder mit kleinem Durchmesser (618) an einem äußeren Ende des Abgabearms (504) beinhaltet; und
einen Konvergenzarm (510), der einen Konvergenzarmschaft (620) aufweist, wobei
der Konvergenzarm (510) einen Verbindungsanschluss (630) beinhaltet, der angepasst ist, um einen Schlauch mit mehreren Hohlräumen (108) aufzunehmen;
der Abgabearmschaft (604) in Fluidverbindung mit dem Konvergenzarmschaft (620) ist, und
der Saugarmschaft (608) in Fluidverbindung mit dem Konvergenzarmschaft (620) und dem Abgabearmschaft (604) ist, wobei der innere ringförmige Hals (640) einen Verbindungskanal (642) in dem Konvergenzarm (510) definiert.

2. Y-Steckverbinder (106) nach Anspruch 1, wobei der Y-Steckverbinder (106) einen inneren Kern (610) beinhaltet, der aus einem ersten Material gefertigt ist, und eine Überschicht (614), die aus einem zweiten Material gefertigt ist, wobei die Überschicht (614) mindestens einen Abschnitt des Y-Steckverbinderkörperabschnitts einschließt.

3. Y-Steckverbinder (106) nach Anspruch 2, wobei die Überschicht (614) mindestens teilweise den inneren Kern (610) einschließt; und/ oder
wobei das erste Material ein starres Kunststoffmaterial ist; und/ oder
wobei die Überschicht (614) eines zweiten Materials aus einem biegsamen Material gefertigt ist; und/ oder
wobei das erste Material ein transparentes Copolyester ist, und wobei das zweite Material weiter ein durchscheinendes thermoplastisches Elastomer ist.

4. Y-Steckverbinder (106) nach Anspruch 1, wobei der Außendurchmesser des sich verjüngenden Hohlschaftsteckverbinders (318a, b) schrittweise abnimmt; oder
wobei der Außendurchmesser des sich verjüngenden Hohlschaftsteckverbinders (318a, b) in konstanter linearer Form abnimmt.

5. Y-Steckverbinder (106) nach Anspruch 1, wobei der Verbindungsanschluss (630) des Konvergenzarmes (510) angepasst ist, um einen Schlauch mit mehreren Hohlräumen (108) in einem Bereich von Größe 14 französisch bis Größe 18 französisch aufzunehmen.

6. Y-Steckverbinder (106) nach Anspruch 1, weiter eine erste Kappe (540) umfassend, die konfiguriert ist, um den Abgabearm (504) abzudichten, und eine zweite Kappe (562), die konfiguriert ist, um den sich verjüngenden Hohlschaftsteckverbinder (318a, b) an dem Saugarm (508) abzudichten;
wobei die erste Kappe (540) vorzugsweise mit dem Abgabearm (504) mit einer ersten Leine (532) gekoppelt ist, die konfiguriert ist, um die erste Kappe (540) umfänglich um den Abgabearm (504) herum zu drehen, und die zweite Kappe (562) mit dem Saugarm (508) mit einer zweiten Leine (532) gekoppelt ist, die konfiguriert ist, um die zweite Kappe (562) umfänglich um den Saugarm (508) herum zu drehen.

7. Y-Steckverbinder (106) nach Anspruch 6, wobei die zweite Kappe (562) angepasst ist, um über die gesamte Oberfläche des sich verjüngenden Hohlschaftsteckverbinders (318a, b) zu passen; oder
wobei die zweite Kappe (562) angepasst ist, um über einen Endabschnitt des sich verjüngenden Hohlschaftsteckverbinders (318a, b) zu passen; oder
wobei die zweite Kappe (562) einen Stopfen (560) beinhaltet, der angepasst ist, um reibschlüssig in den hohlen Abschnitt des äußeren Endes des sich verjüngenden Hohlschaftsteckverbinders (318a, b) zu passen.

8. Magen-Schlauchsystem (104), umfassend:
den Y-Steckverbinder (106) nach Anspruch 1; und
einen Schlauch mit mehreren Hohlräumen (108), der ein offenes proximales Ende (310) und ein geschlossenes distales Ende (314) aufweist, wobei
das offene proximale Ende (310) des Schlauches mit mehreren Hohlräumen (108) angepasst ist, um mit dem Verbindungsanschluss (630) an den Konvergenzarm (510) des Y-Steckverbinders (106) gekoppelt zu werden,
der Schlauch mit mehreren Hohlräumen (108) einen ersten Hohlraum (320) und einen zweiten Hohlraum (324) beinhaltet, die durch eine Wand (330) getrennt sind,
sich der erste Hohlraum (320) von dem proximalen Ende (310) des Schlauches mit mehreren Hohlräumen (108) zu dem distalen Ende (314) des Schlauches mit mehreren Hohlräumen (108) erstreckt,
der erste Hohlraum (320) ein offenes proximales Ende (340) und ein distales Ende (344) aufweist, wobei das distale Ende (344) eine Vielzahl von Öffnungen (360) aufweist,
der zweite Hohlraum (324) ein proximales Ende (350) und ein distales Ende (354) aufweist, wobei das distale Ende (354) des zweiten Hohlraumes (324) in Fluidverbindung mit dem distalen Ende (344) des ersten Hohlraumes (320) ist und eine Fluidverbindungsöffnung (370a) an der Wand (330) zwischen dem ersten Hohlraum (320) und dem zweiten Hohlraum (324) aufweist, die angepasst ist, um es Luft zu ermöglichen, in das distale Ende (344) des ersten Hohlraumes (320) einzutreten,
sich das proximale Ende (350) des zweiten Hohlraumes (324) über das proximale Ende (340) des ersten Hohlraumes (320) hinaus erstreckt, und
das proximale Ende (350) des zweiten Hohlraumes (324) in Fluidverbindung mit der Umgebungsluft ist.

9. Magen-Schlauchsystem (104) nach Anspruch 8, wobei das proximale Ende (350) des zweiten Hohlraumes (324) einen erweiterten Durchmesser aufweist; und/ oder
weiter einen Positionierungsdorn (586) umfassend, der ein erstes Ende (592) und ein zweites Ende (598) aufweist, wobei
der Positionierungsdorn (586) eine Verbindungsvorrichtung (594) beinhaltet, die angepasst ist, um mit dem Steckverbinder mit kleinem Durchmesser (618) des Abgabearmes (504) gekoppelt zu werden,
das erste Ende (592) des Positionierungsdorns (586) einen elektrischen Steckverbinder (590) beinhaltet,
das zweite Ende (598) des Positionierungsdorns (586) eine Spule (596) beinhaltet, die angepasst ist, um sich durch den ersten Hohlraum (320) des Schlauches mit mehreren Hohlräumen (108) zu bewegen,
und
das zweite Ende (598) des Positionierungsdorns (586) innerhalb des ersten Hohlraumes (320) des Schlauches mit mehreren Hohlräumen (108) an dem distalen Ende (344) des ersten Hohlraumes (320) gelegen ist.

10. Magen-Schlauchsystem nach Anspruch 8, weiter ein rückflussverhinderndes Ventil (110) umfassend, das an dem proximalen Ende (350) des zweiten Hohlraumes (324) befestigt ist.

11. Magen-Schlauchsystem nach Anspruch 10, wobei das rückflussverhindernde Ventil (110) ein oberes Ende (810) und ein unteres Ende (820) aufweist, wobei das rückflussverhindernde Ventil (110) beinhaltet:
ein Einwegventil, das angepasst ist, um es dem Fluid zu erlauben, in einer Richtung durch das rückflussverhindernde Ventil (110) hindurch von dem oberen Ende (810) des rückflussverhindernden Ventils (110) zu dem zweiten Hohlraum (324) zu fließen;
oder
das rückflussverhindernde Ventil (110) beinhaltet:
ein rückflussverhinderndes Filtergehäuse (302), das eine Membran aufweist; und
einen Steckverbinder (304), wobei
der Steckverbinder (304) angepasst ist, um das rückflussverhindernde Filtergehäuse (302) aufzunehmen, und
die Membran angepasst ist, um den Fluidfluss zu behindern, während es der Luft erlaubt wird, in beide Richtungen zu fließen.

12. System nach Anspruch 11, wobei das obere Ende (810) des rückflussverhindernden Ventils (110) angepasst ist, um mit einer Spritze gekoppelt zu werden.

13. Enterales Ernährungs- und Saugsystem, umfassend:
eine Fluidquelle (144, 204);
eine Sauganordnung (112), die ein Vakuum (116) und einen Saugbehälter (114) aufweist, wobei das Vakuum (116) und der Saugbehälter (114) durch einen Abschnitt eines Vakuumschlauches (118) verbunden sind;
den Y-Steckverbinder (106) nach Anspruch 1;
einen Schlauch mit mehreren Hohlräumen (108), der ein proximales Ende (310) und ein distales Ende (314) aufweist, wobei
der Schlauch mit mehreren Hohlräumen (108) einen ersten Hohlraum (320) und einen zweiten Hohlraum (324) beinhaltet,
der erste Hohlraum (320) ein proximales Ende (340) und ein distales Ende (344) aufweist, wobei das distale Ende (344) mindestens eine Öffnung (360) aufweist,
das proximale Ende (340) des ersten Hohlraumes (320) angepasst ist, um an dem Verbindungsanschluss (630) des Konvergenzarmes (510) des Y-Steckverbinders (106) befestigt zu werden,
der zweite Hohlraum (324) ein proximales Ende (350) und ein distales Ende (354) aufweist, wobei das distale Ende (354) des zweiten Hohlraumes (324) in Fluidverbindung mit dem distalen Ende (344) des ersten Hohlraumes (320) ist und eine Öffnung (370a) in einer Wand (330) zwischen dem ersten Hohlraum (320) und zweiten Hohlraum (324) aufweist, die angepasst ist, um es Luft zu erlauben, in das distale Ende (344) des ersten Hohlraumes (320) einzutreten,
wobei sich das proximale Ende (350) des zweiten Hohlraumes (324) über das proximale Ende (340) des ersten Hohlraumes (320) hinaus erstreckt, und
sich das proximale Ende (350) des zweiten Hohlraumes (324) zur Umgebungsluft öffnet und einen erweiterten Durchmesser aufweist;
ein Verabreichungsschlauchsegment (150, 212), wobei das Verabreichungsschlauchsegment (150, 212) ein erstes Ende aufweist, das mit der Fluidquelle (144, 204) verbunden ist, und ein zweites Ende, das angepasst ist, um den Abgabearm (504) des Y-Steckverbinders (106) zu verbinden; und
ein Saugrohrsegment (120), wobei
das Saugrohrsegment (120) ein erstes Ende und ein zweites Ende aufweist,
das erste Ende des Saugrohrsegments (120) mit dem Saugbehälter (114) verbunden ist, und
das zweite Ende des Saugrohrsegments (120) angepasst ist, um mit dem sich verjüngenden Hohlschaftsteckverbinder (318a, b) an dem Saugarm (508) verbunden zu werden.

14. System nach Anspruch 13, weiter eine enterale Ernährungspumpe (216) umfassend; und/ oder
weiter ein rückflussverhinderndes Ventil (110) umfassend, das an dem proximalen Ende (350) des zweiten Hohlraumes (324) befestigt ist.

15. Enterales Ernährungs- und Magendruckreliefsystem, umfassend:
einen Magenmaterial-Sammelbehälter (230), wobei der Magenmaterial-Sammelbehälter (230) angepasst ist, um Rückfluss-Fluide aus dem Magen eines Patienten zu sammeln;
eine Fluidquelle (144, 204);
den Y-Steckverbinder (106) nach Anspruch 1;
einen Schlauch mit mehreren Hohlräumen (108), der ein proximales Ende (310) und ein distales Ende (314) aufweist, wobei
der Schlauch mit mehreren Hohlräumen (108) einen ersten Hohlraum (320) und einen zweiten Hohlraum (324) beinhaltet,
der erste Hohlraum (320) ein proximales Ende (340) und ein distales Ende (344) aufweist, wobei das distale Ende (344) mindestens eine Öffnung (360) aufweist,
das proximale Ende des ersten Hohlraumes (320) angepasst ist, um an dem Verbindungsanschluss (630) des Konvergenzarmes (510) des Y-Steckverbinders (106) befestigt zu werden,
der zweite Hohlraum (324) ein proximales Ende (350) und ein distales Ende (354) aufweist, wobei das distale Ende (354) des zweiten Hohlraumes (324) in Fluidverbindung mit dem distalen Ende (344) des ersten Hohlraumes (320) ist und eine Fluidverbindungsöffnung (370a) an einer Wand (330) zwischen dem ersten Hohlraum (320) und dem zweiten Hohlraum (324) aufweist, die angepasst ist, um es Luft zu ermöglichen, in das distale Ende (344) des ersten Hohlraumes (320) einzutreten,
sich das proximale Ende (350) des zweiten Hohlraumes (324) über das proximale Ende (340) des ersten Hohlraumes (320) hinaus erstreckt, und
sich das proximale Ende (350) des zweiten Hohlraumes (324) zur Umgebungsluft öffnet und einen erweiterten Durchmesser aufweist;
ein Verabreichungsschlauchsegment (150, 212), wobei das Verabreichungsschlauchsegment (150, 212) ein erstes Ende aufweist, das mit der Fluidquelle (144, 204) verbunden ist, und ein zweites Ende, das angepasst ist, um den Abgabearm (504) des Y-Steckverbinders (106) zu verbinden; und
ein Reliefschlauchsegment (240), wobei
das Reliefschlauchsegment (240) ein erstes Ende und ein zweites Ende aufweist, und
das erste Ende des Reliefschlauchsegments (240) mit dem Magenmaterial-Sammelbehälter (230) verbunden ist, und das zweite Ende des Reliefschlauchsegments (240) mit dem offenen proximalen Ende (350) des zweiten Hohlraumes (324) des Schlauches mit mehreren Hohlräumen (108) verbunden ist; und
weiter vorzugsweise einen rückflussverhindernden Ventilsteckverbinder (304) umfassend, der an dem proximalen Ende (350) des zweiten Hohlraumes (324) befestigt ist.

## Revendications

1. Raccord en Y (106) destiné à être utilisé dans un système gastrique (104) comprenant:
un bras d'aspiration (508) présentant une tige de bras d'aspiration (608) et une extrémité extérieure, dans lequel
la tige de bras d'aspiration (608) présente une extrémité proximale (690) et une extrémité distale (692),
le bras d'aspiration (508) inclut un raccord de tige creux conique (318a, b) adapté pour s'ajuster par frottement dans une section de tubulure (120),
le raccord de tige creux conique (318a, b) présente une base et une extrémité de sortie (694),
le raccord de tige creux conique (318a, b) est aligné axialement avec la tige de bras d'aspiration (608) et la base du raccord de tige creux (318a, b) est couplée à l'extrémité extérieure du bras d'aspiration (508), et
le raccord de tige creux conique (318a, b) présente un diamètre extérieur qui diminue à partir de la base du raccord de tige creux (318a, b) jusqu'à l'extrémité de sortie (694) du raccord de tige creux (318a, b) ;
une valve unidirectionnelle (650, 650a) interposée entre l'extrémité de sortie (694) du raccord de tige creux (318a, b) et l'extrémité proximale (690) de la tige de bras d'aspiration (608) adaptée pour permettre à du fluide de s'écouler dans une direction à travers la tige de bras d'aspiration (608) à partir de la base du raccord de tige creux (318a, b) vers l'extrémité de sortie (694) du raccord de tige creux (318a, b) ;
un bras de distribution (504) présentant une tige de bras de distribution (604), dans lequel le bras de distribution (504) inclut un raccord fileté à petit alésage (618) à une extrémité extérieure du bras de distribution (504) ; et
un bras de convergence (510) présentant une tige de bras de convergence (620), dans lequel
le bras de convergence (510) inclut un orifice de raccordement (630) adapté pour recevoir un tube à lumières multiples (108),
la tige de bras de distribution (604) est en communication fluidique avec la tige de bras de convergence (620), et
la tige de bras d'aspiration (608) est en communication fluidique avec la tige de bras de convergence (620) et la tige de bras de distribution (604), dans lequel un col annulaire intérieur (640) définit un canal de liaison (642) dans le bras de convergence (510).

2. Raccord en Y (106) selon la revendication 1, dans lequel le raccord en Y (106) inclut un noyau interne (610) constitué d'un premier matériau et une surcouche (614) constituée d'un second matériau, la surcouche (614) renfermant au moins une partie de la partie de corps de raccord en Y.

3. Raccord en Y (106) selon la revendication 2, dans lequel la surcouche (614) renferme au moins partiellement le noyau interne (610) ; et/ou
dans lequel le premier matériau est un matériau plastique rigide ; et/ou
dans lequel la surcouche de second matériau (614) est réalisée à partir d'un matériau pliable ; et/ou
dans lequel le premier matériau est un copolyester transparent et dans lequel en outre le second matériau est un élastomère thermoplastique translucide.

4. Raccord en Y (106) selon la revendication 1, dans lequel le diamètre extérieur du raccord de tige creux conique (318a, b) diminue d'une manière progressive ; ou
dans lequel le diamètre extérieur du raccord de tige creux conique (318a, b) diminue d'une manière linéaire constante.

5. Raccord en Y (106) selon la revendication 1, dans lequel l'orifice de raccordement (630) du bras de convergence (510) est adapté pour recevoir un tube à lumières multiples (108) allant d'une taille de 14 unités Charrière à une taille de 18 unités Charrière.

6. Raccord en Y (106) selon la revendication 1, comprenant en outre un premier capuchon (540) configuré pour sceller le bras de distribution (504) et un second capuchon (562) configuré pour sceller le raccord de tige creux conique (318a, b) sur le bras d'aspiration (508) ;
de préférence dans lequel le premier capuchon (540) est couplé au bras de distribution (504) avec une première attache (532) configurée pour faire tourner circonférentiellement le premier capuchon (540) autour du bras de distribution (504) et le second capuchon (562) est couplé au bras d'aspiration (508) avec une seconde attache (532) configurée pour faire tourner circonférentiellement le second capuchon (562) autour du bras d'aspiration (508).

7. Raccord en Y (106) selon la revendication 6, dans lequel le second capuchon (562) est adapté pour s'ajuster au-dessus de toute la surface du raccord de tige creux conique (318a, b) ; ou
dans lequel le second capuchon (562) est adapté pour s'ajuster au-dessus d'une partie d'extrémité du raccord de tige creux conique (318a, b) ; ou
dans lequel le second capuchon (562) inclut un bouchon (560) adapté pour s'ajuster par frottement à l'intérieur de la partie creuse de l'extrémité extérieure du raccord de tige creux conique (318a, b).

8. Système de tubulure gastrique (104), comprenant :
un raccord en Y (106) selon la revendication 1, et
un tube à lumières multiples (108) présentant une extrémité proximale ouverte (310) et une extrémité distale fermée (314), dans lequel
l'extrémité proximale ouverte (310) du tube à lumières multiples (108) est adaptée pour se coupler à l'orifice de raccordement (630) sur le bras de convergence (510) du raccord en Y (106),
le tube à lumières multiples (108) inclut une première lumière (320) et une seconde lumière (324) qui sont séparées par une paroi (330),
la première lumière (320) s'étend depuis l'extrémité proximale (310) du tube à lumières multiples (108) vers l'extrémité distale (314) du tube à lumières multiples (108),
la première lumière (320) présente une extrémité proximale ouverte (340) et une extrémité distale (344), l'extrémité distale (344) présente une pluralité d'ouvertures (360),
la seconde lumière (324) présente une extrémité proximale (350) et une extrémité distale (354), l'extrémité distale (354) de la seconde lumière (324) est en communication fluidique avec l'extrémité distale (344) de la première lumière (320) et présente une ouverture de communication fluidique (370a) sur la paroi (330) entre la première lumière (320) et la seconde lumière (324) adaptée pour permettre à de l'air d'entrer dans l'extrémité distale (344) de la première lumière (320),
l'extrémité proximale (350) de la seconde lumière (324) s'étend au-delà de l'extrémité proximale (340) de la première lumière (320), et
l'extrémité proximale (350) de la seconde lumière (324) est en communication fluidique avec l'air ambiant.

9. Système de tubulure gastrique (104) selon la revendication 8, dans lequel l'extrémité proximale (350) de la seconde lumière (324) présente un diamètre agrandi ; et/ou
comprenant en outre un stylet de positionnement (586) présentant une première extrémité (592) et une seconde extrémité (598), dans lequel
le stylet de positionnement (586) inclut un dispositif d'union (594) adapté pour être couplé au raccord à petit alésage (618) du bras de distribution (504),
la première extrémité (592) du stylet de positionnement (586) inclut un raccord électrique (590),
la seconde extrémité (598) du stylet de positionnement (586) inclut une bobine (596) qui est adaptée pour se déplacer à travers la première lumière (320) du tube à lumières multiples (108), et
la seconde extrémité (598) du stylet de positionnement (586) est située à l'intérieur de la première lumière (320) du tube à lumières multiples (108) au niveau de l'extrémité distale (344) de la première lumière (320).

10. Système de tubulure gastrique selon la revendication 8, comprenant en outre une valve anti-reflux (110) fixée à l'extrémité proximale (350) de la seconde lumière (324).

11. Système de tubulure gastrique selon la revendication 10, dans lequel la valve anti-reflux (110) présente une extrémité supérieure (810) et une extrémité inférieure (820), la valve anti-reflux (110) incluant :
une valve unidirectionnelle adaptée pour permettre à du fluide de s'écouler dans une direction à travers la valve anti-reflux (110) depuis l'extrémité supérieure (810) de la valve anti-reflux (110) jusqu'à la seconde lumière (324) ; ou
la valve anti-reflux (110) incluant :
un boîtier de filtre anti-reflux (302) présentant une membrane ; et
un raccord (304), dans lequel
le raccord (304) est adapté pour recevoir le boîtier de filtre anti-reflux (302), et
la membrane est adaptée pour obstruer un écoulement de fluide tout en permettant à de l'air de s'écouler dans les deux directions.

12. Système selon la revendication 11, dans lequel l'extrémité supérieure (810) de la valve anti-reflux (110) est adaptée pour être couplée à une seringue.

13. Système d'alimentation entérale et d'aspiration comprenant :
une source de fluide (144, 204) ;
un ensemble d'aspiration (112) présentant un vide (116) et une cartouche d'aspiration (114), dans lequel le vide (116) et la cartouche d'aspiration (114) sont raccordés par une section de tubulure à vide (118) ;
le raccord en Y (106) selon la revendication 1 ;
un tube à lumières multiples (108) présentant une extrémité proximale (310) et une extrémité distale (314), dans lequel
le tube à lumières multiples (108) inclut une première lumière (320) et une seconde lumière (324),
la première lumière (320) présente une extrémité proximale (340) et une extrémité distale (344), l'extrémité distale (344) présente au moins une ouverture (360),
l'extrémité proximale (340) de la première lumière (320) étant adaptée pour se fixer à l'orifice de raccordement (630) sur le bras de convergence (510) du raccord en Y (106),
la seconde lumière (324) présentant une extrémité proximale (350) et une extrémité distale (354), l'extrémité distale (354) de la seconde lumière (324) en communication fluidique avec l'extrémité distale (344) de la première lumière (320) et présentant une ouverture (370a) sur une paroi (330) entre la première lumière (320) et la seconde lumière (324) adaptée pour permettre à de l'air d'entrer dans l'extrémité distale (344) de la première lumière (320),
l'extrémité proximale (350) de la seconde lumière (324) s'étendant au-delà de l'extrémité proximale (340) de la première lumière (320), et
l'extrémité proximale (350) de la seconde lumière (324) ouverte à l'air ambiant et présentant un diamètre agrandi ;
un segment de tubulure d'administration (150, 212), dans lequel le segment de tubulure d'administration (150, 212) présente une première extrémité raccordée à la source de fluide (144, 204) et une seconde extrémité adaptée pour se raccorder au bras de distribution (504) du raccord en Y (106) ; et
un segment de tubulure d'aspiration (120), dans lequel
le segment de tubulure d'aspiration (120) présente une première extrémité et une seconde extrémité,
la première extrémité du segment de tubulure d'aspiration (120) est raccordée à la cartouche d'aspiration (114), et
la seconde extrémité du segment de tubulure d'aspiration (120) est adaptée pour se raccorder au raccord de tige creux conique (318a, b) sur le bras d'aspiration (508).

14. Système selon la revendication 13, comprenant en outre une pompe d'alimentation entérale (216) ; et/ou
comprenant en outre une valve anti-reflux (110) fixée à l'extrémité proximale (350) de la seconde lumière (324).

15. Système d'alimentation entérale et de décharge de pression gastrique comprenant :
un réservoir de collecte de matière gastrique (230), dans lequel le réservoir de collecte de matière gastrique (230) est adapté pour collecter des fluides de reflux à partir d'un estomac de patient ;
une source de fluide (144, 204) ;
le raccord en Y (106) selon la revendication 1 ;
un tube à lumières multiples (108) présentant une extrémité proximale (310) et une extrémité distale (314), dans lequel
le tube à lumières multiples (108) inclut une première lumière (320) et une seconde lumière (324),
la première lumière (320) présente une extrémité proximale (340) et une extrémité distale (344), l'extrémité distale (344) présente au moins une ouverture (360),
l'extrémité proximale de la première lumière (320) étant adaptée pour se fixer à l'orifice de raccordement (630) sur le bras de convergence (510) du raccord en Y (106),
la seconde lumière (324) présentant une extrémité proximale (350) et une extrémité distale (354), l'extrémité distale (354) de la seconde lumière (324) en communication fluidique avec l'extrémité distale (344) de la première lumière (320) et présentant une ouverture (370a) sur une paroi (330) entre la première lumière (320) et la seconde lumière (324) adaptée pour permettre à de l'air d'entrer dans l'extrémité distale (344) de la première lumière (320),
l'extrémité proximale (350) de la seconde lumière (324) s'étendant au-delà de l'extrémité proximale (340) de la première lumière (320), et
l'extrémité proximale (350) de la seconde lumière (324) ouverte à l'air ambiant et présentant un diamètre agrandi ;
un segment de tubulure d'administration (150, 212), dans lequel le segment de tubulure d'administration (150, 212) présente une première extrémité raccordée à la source de fluide (144, 204) et une seconde extrémité adaptée pour se raccorder au bras d'aspiration (508) du raccord en Y (106) ; et
un segment de tubulure de décharge (240), dans lequel
le segment de tubulure de décharge (240) présente une première extrémité et une seconde extrémité, et
la première extrémité du segment de tube de décharge (240) est raccordée au réservoir de collecte de matière gastrique (230), et la seconde extrémité du segment de tube de décharge (240) est raccordée à l'extrémité proximale ouverte (350) de la seconde lumière (324) du tube à lumières multiples (108); et
comprenant en outre de préférence un raccord de valve anti-reflux (304) fixé à l'extrémité proximale (350) de la seconde lumière (324).
